# EUROPEAN PATENT APPLICATION

(11) **EP 4 316 487 A1**
(43) Date of publication of application: **07.02.2024**
(21) Application number: 22781539.6
(22) Date of filing: 28.03.2022
(51) Int. Cl.: A61K 31/423, A61P 25/28, A23L 33/10

(54) **COMPOSITION FOR PREVENTING OR TREATING NEURODEGENERATIVE DISEASE COMPRISING COMPOUND INDUCING EXPRESSION OF ANTI-AGING GENE KLOTHO**

(30) Priority: 01.04.2021 KR 20210042958; 30.09.2021 KR 20210130307
(71) Applicant: Klotho Sciences, Gyeonggi-do 13605 (KR)
(72) Inventor: JUNG, Dong Ju, Cheonan-si, Chungcheongnam-do 31166 (KR)
(74) Representative: Dehns
(86) International application number: PCT/KR2022/004341
(87) International publication number: WO 2022/211420

(57) **Abstract**

The present invention relates to a composition for the prevention or treatment of neurodegenerative disease, comprising compounds that induce the expression of the anti-aging gene klotho. The compounds represented by Chemical Formula 1 according to the invention are highly effective in enhancing the expression of the Klotho gene, a gene associated with aging, and thus can be useful for a pharmaceutical composition or a food composition for the prevention, amelioration or treatment of neurodegenerative disease.

## Description

### [Technical Field]

This application claims benefit of priority based on Korean Patent Application No. 10-2021-0042958 filed on April 1, 2021 and Korean Patent Application No. 10-2021-0130307 filed on September 30, 2021, the disclosures of which are incorporated herein by reference in their entireties.

The present invention relates to a composition for the prevention or treatment of neurodegenerative disease comprising a compound that induces the expression of the anti-aging gene klotho.

### [Background Art]

Dysregulation of the neuroimmune system has been implicated in the pathogenesis of a variety of neurodegenerative diseases, and many studies have reported neuroinflammatory responses within the central nervous system, including Parkinson's disease, Alzheimer's disease, traumatic injury, severe stroke, and seizure-induced brain injury.

On the other hand, the fact that there may be genes capable of regulating aging in animals became known through senescence-accelerated mice (SAM) reported in 1981. These mice, which were accidentally made while crossing AKR/J series mice, aged much faster than mice of the same family, and these mice were confirmed to have mutations in several genes. Later, the discovery of a single group of aging-related genes was reported in the 1990s. The reported genes were genes that expressed an enzyme called DNA helicase in the RecQ family. It has been reported that, when mutations occur in these genes, the result that premature aging occurs or cancer is produced, which is known to be caused by affecting DNA repair. A single gene associated with aging is the klotho gene, which was reported in 1997. The klotho gene was accidentally discovered while creating an animal model for transgenic hypertension mice, but in mice that could not express this gene, premature aging occurred and their lives were shortened. More interestingly, the lifespan of mice that later increased the expression of this gene increased by 20.0 to 30.8% in males and 18.8 to 19.0% in females. This became the first opportunity to inform the world that the lifespan of mice can be increased or decreased depending on the expression of a single gene. In addition, the base sequence of the klotho gene was very similar among animals, and it was reported that it was about 98% identical between mice and humans. This indicates that lifespan can be regulated according to the expression of the klotho gene in humans as well.

In humans, α-klotho (hereafter referred to as klotho), a member of the klotho gene family known to be associated with aging, is located on chromosome 13 and produces a membrane protein with sequence similarity to β-glucosidase. The protein klotho is mainly expressed in renal tubular epithelial cells and brain choroid plexus, and has been reported to be expressed in some parathyroid glands. The klotho gene is a gene associated with various aging phenotypes, and in mice lacking the klotho gene, a syndrome similar to the aging process such as shortened lifespan, decreased activity, growth retardation, atherosclerosis, arterial calcification, osteoporosis, immature reproductive organs, infertility, skin atrophy, and emphysema occurs. In klotho mutant mice, atherosclerosis similar to Monckeberg type arteriosclerosis caused by aging in humans is observed in all arteries from the aorta to the arterioles, and angiogenesis and vasculogenesis are impaired.

The expression of klotho mRNA is significantly higher in kidney tissue than in other tissues, but the expression of klotho mRNA is decreased in the kidneys of mice in a disease model of hypertension, type 2 diabetes, diabetic nephropathy, and chronic renal failure. In mice with reduced klotho expression, the production of nitric oxide, which is a relaxation factor derived from vascular endothelium, decreases, and when the klotho gene is injected into Otsuka Long-Evans Tokushima fatty rat (OLETF) mice, which simultaneously have risk factors for many cardiovascular diseases, using a viral gene carrier, endovascular dysfunction is alleviated, the production of NO is increased, and blood pressure is lowered by suppressing vascular thickening and fibrosis. In addition, the klotho gene also affects glucose and insulin metabolism in mice, and statin, which is a representative therapeutic agent for hypercholesterolemia, increases the expression of klotho mRNA in renal proximal tubule cells. In mice with reduced klotho expression, osteopenia of low bone turnover states occurs due to impaired differentiation of both osteoblasts and osteoclasts, which is similar to the characteristics of bone loss and senile osteoporosis according to an increase in age in humans. Furthermore, in klotho mutant mice, abnormal elongation of the trabecular bone at the epiphysis area and abnormal trabecular bone tissue on micro-computerized tomography are observed, which is due to the disorder of the bone resorption process. Changes in clinical phenotypes caused by mutations in the klotho gene observed in humans are diverse. The functional variant of klotho (KL-VS) modification with mutations in three sites of the klotho gene exon2 is associated with lipid metabolism, blood pressure, lifespan, cognitive function, coronary artery disease and cerebrovascular disease, and microsatellite polymorphism and single base genetic polymorphism of the klotho gene are associated with bone density, and it has also been reported that the single base genetic polymorphism of the klotho gene in healthy adult women is associated with cardiovascular disease risk factors and bone density. Recently, the association between the klotho gene and Alzheimer's disease has also been reported in several papers. In Alzheimer dementia mouse models, it has been reported that overexpression of klotho increases the lifespan of mice by 30% and inhibits a reduction in cognitive function. Furthermore, it was observed that the production of amyloid beta protein in the brain was reduced by 50% by klotho expression. In humans, the expression level of klotho is inversely proportional to the progression of Alzheimer's disease, and a report was submitted that the klotho protein reduces the amount of inflammatory cytokines in the blood of patients with Alzheimer's disease.

Efforts have been continuously made to develop a material capable of inducing the expression of the klotho gene, which has such a clear senescence inhibitory effect. Among known materials, those reported to be able to induce the expression of klotho include rapamycin, vitamin D, statin, and the like. In 2012, a research team at Boston University reported three compounds that they found by screening for compounds capable of inducing the expression of the klotho gene using a library of 150,000 compounds in total.

The present inventors selected a compound called compound H, which has a structure that is highly likely to be developed as a pharmaceutical, among the compounds, confirmed through actual experiments that the compound can express the klotho gene in cells, and published the research results on its mechanism of action in a paper. Since then, the present inventors have conducted experiments to analyze the structure of compound H (Comparative Example 1), found the structural characteristics of the compound capable of inducing klotho expression, and based on this, made a new compound whose activity was increased by 10 times or more. In addition, the present inventors experimentally confirmed that the present novel compounds inducing the expression of the anti-aging gene klotho are useful for preventing or treating neurodegenerative disease, thereby completed the present invention.

### [Disclosure]

### [Technical Problem]

An object of the present invention is to provide a pharmaceutical composition for preventing or treating neurodegenerative disease comprising a compound represented by Chemical Formula 1 or a pharmaceutically acceptable salt thereof.

Another object of the present invention is to provide a health functional food composition for preventing or improving neurodegenerative disease, or a food composition comprising the compound represented by Chemical Formula 1 or a pharmaceutically acceptable salt thereof.

### [Technical Solution]

To achieve the objects, the present invention provides a pharmaceutical composition for preventing or treating neurodegenerative disease comprising a compound represented by Chemical Formula 1 below or a pharmaceutically acceptable salt thereof as an active ingredient: 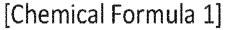 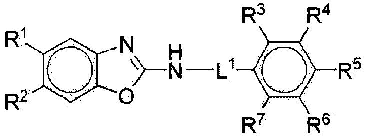 (in Chemical Formula 1,
L¹ is a single bond or 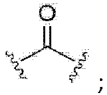
R¹ and R² are each -H, -OH, a C₁₋₁₀ straight or branched alkyl, or a C₆₋₈ arylamide, wherein the aryl of the arylamide may be substituted with one or more of a halogen, -NO₂ and a C₁₋₁₀ straight or branched alkyl halide;
R¹ and R² may form a C₆₋₈ aryl with a carbon atom to which they are linked; and
R³, R⁴, R⁵, R⁶ and R⁷ are each -H, a halogen, -NO₂ or a C₁₋₁₀ straight or branched alkyl).

In addition, the present invention provides a health functional food composition for preventing or improving neurodegenerative disease comprising the compound represented by Chemical Formula 1 or a pharmaceutically acceptable salt thereof as an active ingredient.

Furthermore, the present invention provides a food composition for preventing or improving neurodegenerative disease comprising the compound represented by Chemical Formula 1 or a pharmaceutically acceptable salt thereof as an active ingredient.

In addition, the present invention provides a method for preventing or treating neurodegenerative disease comprising the step of administering or ingesting a composition containing the compound represented by Chemical Formula 1 or a pharmaceutically acceptable salt thereof as an active ingredient to a subject.

In addition, the present invention provides a use for preventing or treating neurodegenerative disease of a composition comprising the compound represented by Chemical Formula 1 or a pharmaceutically acceptable salt thereof as an active ingredient.

### [Advantageous Effects]

The compound represented by Chemical Formula 1 according to the present invention has an excellent effect of improving the expression level of klotho gene, which is a gene related to aging, and can be usefully used as a pharmaceutical composition or food composition for preventing, improving or treating neurodegenerative disease.

### [Description of Drawings]

FIG. 1A illustrates the results of luciferase expression experiments using a reporter gene including a promoter from the start site of the human klotho gene of Comparative Examples 1 to 4 to the front of 1.7 kbp.
FIG. 1B illustrates the results of luciferase expression experiments using a reporter gene including a promoter from the start site of the human klotho gene of Comparative Examples 1 to 4 to the front of 240 bp.
FIG. 2 illustrates the results of luciferase expression experiments using a reporter gene including a promoter included up to -2.1 kb upstream of the human klotho gene of Examples 1 to 6.
FIG. 3 illustrates the results of luciferase expression experiments using a reporter gene including a promoter included up to -2.1 kb upstream of the human klotho gene of Examples 1 to 3.
FIG. 4 is the result of confirming the mRNA expression level of the klotho (KL) gene in Examples 1 and 2 by RT-PCR.
FIG. 5 is the result of confirming the expression of the klotho gene in RPTEC cells treated with the compounds of Examples 1 and 2 and Examples 7 to 10.
FIG. 6 is the result of confirming cytotoxicity in HK2 cells treated with the compounds of Examples 1, 9 and 10.
FIG. 7 is the result of confirming the neuronal aging inhibitory effect of the KS1 compound (Example 10) on HT22 cells (nerve cells derived from mouse hippocampus).
FIG. 8 is the result of confirming the neuronal inflammation inhibitory effect of the KS1 compound (Example 10) on HT22 cells (nerve cells derived from mouse hippocampus).
FIG. 9 is the result of confirming the effect of reducing the expression of the cognitive dysfunction protein of the KS1 compound (Example 10) in the 5xFAD cognitive dysfunction animal model.
FIG. 10 is the result of confirming the effect of increasing the expression of the neuronal cell marker NeuN of the KS1 compound (Example 10) in the 5xFAD cognitive dysfunction animal model.
FIG. 11 is the result of confirming the effect of KS1 compound (Example 10) on improving object search ability in the 5xFAD cognitive dysfunction animal model.
FIG. 12 is the result of confirming the spatial learning and memory improvement effect of the KS1 compound (Example 10) through a passive avoidance experiment in a 5xFAD cognitive dysfunction animal model.

### [Modes of the Invention]

Hereinafter, the present invention will be described in detail.

The present invention relates to a composition for preventing or treating/improving neurodegenerative disease, comprising a compound represented by Chemical Formula 1 or a pharmaceutically acceptable salt thereof as an active ingredient.

The compounds represented by Chemical Formula 1 below according to the present invention are effective in enhancing the expression of the Klotho gene, a gene associated with aging, and can be usefully used as a pharmaceutical composition or food composition for the prevention, amelioration or treatment of neurodegenerative disease.

### Pharmaceutical composition for preventing or treating neurodegenerative disease

The present invention provides a pharmaceutical composition for preventing or treating neurodegenerative disease comprising a compound represented by Chemical Formula 1 below or a pharmaceutically acceptable salt thereof: 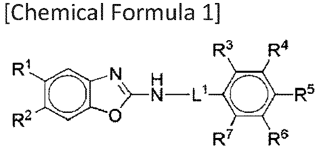 in Chemical Formula 1,
L¹ is a single bond or 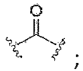
R¹ and R² are each -H, -OH, a C₁₋₁₀ straight or branched alkyl, or a C₆₋₈ arylamide, wherein the aryl of the arylamide may be substituted with one or more of a halogen, -NO₂ and a C₁₋₁₀ straight or branched alkyl halide;
R ¹ and R² may form a C₆₋₈ aryl with a carbon atom to which they are linked; and
R³, R⁴, R⁵, R⁶ and R⁷ may each be -H, a halogen, -NO₂ or a C₁₋₁₀ straight or branched alkyl.

In an exemplary embodiment according to the present invention,
L¹ is a single bond or

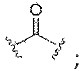

R¹ and R² are each -H, -OH, a C₁₋₅ straight or branched alkyl, or a C₆₋₇ arylamide, wherein the aryl of the arylamide may be substituted with one or more of a halogen, -NO₂ and a C₁₋₅ straight or branched alkyl halide;
R¹ and R² may form a C₆₋₇ aryl with a carbon atom to which they are linked; and
R³, R⁴, R⁵, R⁶ and R⁷ may each be -H, a halogen, -NO₂ or a C₁₋₅ straight or branched alkyl.

In an exemplary embodiment according to the present invention,
L¹ is a single bond or 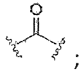
R¹ and R² are each -H, -OH, -CH₃, or phenylamide, wherein the phenyl of the phenylamide may be substituted with one or more of -Cl, -NO₂ and -CH₂Cl;
R¹ and R² may form a phenyl with a carbon atom to which they are linked; and
R³, R⁴, R⁵, R⁶ and R⁷ may each be -H, -F, -Cl, -NO₂ or -CH₂CH₃.

In an exemplary embodiment according to the present invention,
L¹ is a single bond or 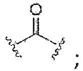
R1 is -H, -OH, -CH₃, 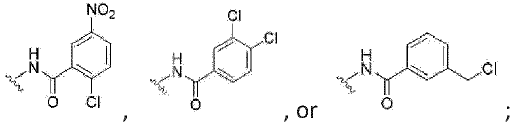
R² is -H;
R¹ and R² may form a phenyl with a carbon atom to which they are linked;
R³ is -H or -Cl;
R⁴ is -H, -F or -Cl;
R⁵ is -F, -Cl, -NO ₂, or -CH₂CH₃;
R⁶ is -H; and
R⁷ may be -H.

Preferred example of the compound represented by Chemical Formula 1 according to the present invention may be the following compound group:
1) N-(benzo[d]oxazol-2-yl)-2-chloro-4-nitrobenzamide;
2) 8-methyl-2-[N-(3,4-dichlorophenyl)]aminobenzoxazole;
3) 2-((3,4-dichlorophenyl)amino)benzo[d]oxazol-5-ol;
4) N-(2-(4-ethylphenylamino)benzo[d]oxazol-5-yl)-2-chloro-5-nitrobenzamide;
5) N-(2-(4-ethylphenylamino)benzo[d]oxazol-5-yl)-3,4-dichlorobenzamide;
6) N-(2-(4-ethylphenylamino)benzo[d]oxazol-5-yl)-3-(chloromethyl)benzamide;
7) 2-[N-(3,4-dichlorophenyl)]aminobenzoxazole;
8) N-(3,4-dichlorophenyl)naphtho[2,3-d]oxazol-2-amine;
9) N-(3,4-difluorophenyl)-5-methylbenzo[d]oxazol-2-amine; or
10) N-(3,4-difluorophenyl)benzo[d]oxazol-2-amine.

The compound represented by Chemical Formula 1 of the present invention can be used in the form of a pharmaceutically acceptable salt, and as a salt, an acid addition salt formed by a pharmaceutically acceptable free acid is useful. The expression "pharmaceutically acceptable salt" refers to any organic or inorganic addition salt of a base compound of Chemical Formula 1 whose concentration has effective action because it is relatively non-toxic and harmless to the patients and whose side effects resulting from the salt do not degrade the beneficial efficacy of the base compound of Chemical Formula 1. These salts may use an inorganic acid and an organic acid as a free acid, as the inorganic acid, it is possible to use hydrochloric acid, bromic acid, nitric acid, sulfuric acid, perchloric acid, phosphoric acid, and the like, and as the organic acid, it is possible to use citric acid, acetic acid, lactic acid, maleic acid, fumaric acid, gluconic acid, methanesulfonic acid, glycolic acid, succinic acid, tartaric acid, galacturonic acid, embonic acid, glutamic acid, aspartic acid, oxalic acid, (D) or (L) malic acid, maleic acid, methanesulfonic acid, ethanesulfonic acid, 4-toluenesulfonic acid, salicylic acid, citric acid, benzoic acid, malonic acid, and the like. Further, these salts include alkali metal salts (sodium salts, potassium salts, and the like), alkaline earth metal salts (calcium salts, magnesium salts, and the like), and the like. For example, as an acid addition salt, acetate, aspartate, benzoate, besylate, bicarbonate/carbonate, bisulfate/sulfate, borate, camsylate, citrate, edisylate, esylate, formate, fumarate, gluceptate, gluconate, glucuronate, hexafluorophosphate, hibenzate, hydrochloride/chloride, hydrobromide/bromide, hydroiodide/iodide, isethionate, lactate, malate, maleate, malonate, mesylate, methyl sulfate, naphthylate, 2-napsylate, nicotinate, nitrate, orotate, oxalate, palmitate, pamoate, phosphate/hydrogen phosphate/dihydrogen phosphate, saccharate, stearate, succinate, tartrate, tosylate, trifluoroacetate, aluminum, arginine, benzathine, calcium, choline, diethylamine, diolamine, glycine, lysine, magnesium, meglumine, olamine, potassium, sodium, tromethamine, zinc salts, and the like may be included, and among them, hydrochloride or trifluoroacetate is preferred.

In addition, the compound represented by Chemical Formula 1 of the present invention includes not only pharmaceutically acceptable salts, but also all salts, isomers, hydrates and solvates that can be prepared by typical methods.

The addition salt according to the present invention may be prepared by a typical method, and may be prepared, for example, by dissolving the compound of Compound Formula 1 in a water-miscible organic solvent, for example, acetone, methanol, ethanol, or acetonitrile, or the like, adding an excessive amount of an organic acid thereto or adding an aqueous acid solution of an inorganic acid thereto, followed by precipitation or crystallization. Subsequently, the acid addition salt may be prepared by evaporating the solvent or excess acid from this mixture, and then drying the mixture or suction-filtering a precipitated salt.

### Preparation method 1 of the compounds

The present invention can provide a method for preparing a compound represented by Chemical Formula 1A, as shown in the following Reaction Scheme 1, the method including:
obtaining Compound 4 by dissolving Compound 2 in an organic solvent, and then adding Compound 3 thereto, and reacting the resulting mixture at 10 to 50°C for 12 to 20 hours (Step 1); and
obtaining Compound 1A by adding an organic solvent in which Compound 4 obtained in Step 1 is dissolved dropwise to an organic solvent in which potassium superoxide is dissolved, and then reacting the resulting mixture at 15 to 30°C for 10 to 16 hours (Step 2).

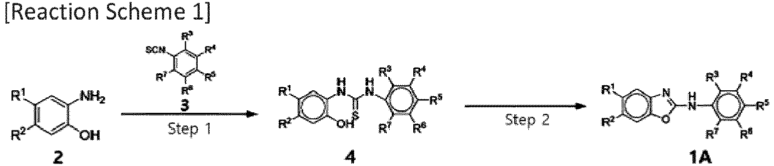

In Reaction Scheme 1,
R¹, R², R³, R⁴, R⁵, R⁶ and R⁷ are the same as defined in Chemical Formula 1 above, and
Compound 1A is included in Chemical Formula 1 above.

In the preparation method of the present invention, as an example of the organic solvent, methanol (MeOH), dimethylformamide (DMF), acetonitrile (MeCN), tetrahydrofuran (THF), dichloromethane (DCM), 1,2-dimethoxyethane, benzene, toluene, xylene, dimethyl sulfoxide (DMSO), or dioxane may be used alone or in mixtures thereof.

In the preparation method of the present invention, an example of the compound that can be prepared by the above preparation method may be 8-methyl-2-[N-(3,4-dichlorophenyl)]aminobenzoxazole, 2-[N-(3,4-dichlorophenyl)]aminobenzoxazole, N-(3,4-dichlorophenyl)naphtho[2,3-d]oxazol-2-amine, N-(3,4-difluorophenyl)-5-methylbenzo[d]oxazol-2-amine or N-(3,4-difluorophenyl)benzo[d]oxazol-2-amine.

### Preparation method 2 of the compounds

The present invention can provide a method for preparing a compound represented by Chemical Formula 1B, as shown in the following Reaction Scheme 2, the method including:
obtaining Compound 6 by dissolving Compound 5 in an organic solvent, and then adding Compound 3 thereto, and reacting the resulting mixture at 10 to 50°C for 12 to 20 hours (Step 1);
obtaining Compound 7 by adding an organic solvent in which Compound 6 obtained in Step 1 is dissolved dropwise to an organic solvent in which potassium superoxide is dissolved, and then reacting the resulting mixture for 12 to 24 hours (Step 2); and
obtaining Compound 1B by dissolving Compound 7 in an organic solvent, adding boron tribromide (BBr₃) thereto, and then reacting the resulting mixture at room temperature for 20 to 28 hours (Step 3).

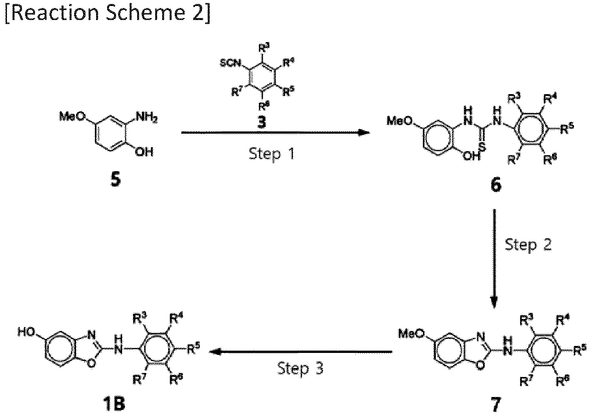

In Reaction Scheme 2,
R³, R⁴, R⁵, R⁶ and R⁷ are the same as defined in Chemical Formula 1 above, and
Compound 1B is included in Chemical Formula 1 above.

In the preparation method of the present invention, as an example of the organic solvent, methanol (MeOH), dimethylformamide (DMF), acetonitrile (MeCN), tetrahydrofuran (THF), dichloromethane (DCM), 1,2-dimethoxyethane, benzene, toluene, xylene, dimethyl sulfoxide (DMSO), or dioxane may be used alone or in combination.

In the preparation method of the present invention, an example of the compound that can be prepared by the above preparation method may be 2-((3,4-dichlorophenyl)amino)benzo[d]oxazol-5-ol.

### Preparation method 3 of the compounds

The present invention provides a method for preparing a compound represented by Chemical Formula 1C, as shown in the following Reaction Scheme 3, the method including:
obtaining Compound 9 by dissolving Compound 8, carbon disulfide, iodomethane and sodium hydride in an organic solvent, and then reacting the resulting solution at 10 to 50°C for 2 to 8 hours (Step 1); and
obtaining Compound 1C by dissolving Compound 9 and Compound 2 in an organic solvent, and then reacting the resulting solution for 2 to 8 hours (Step 2).

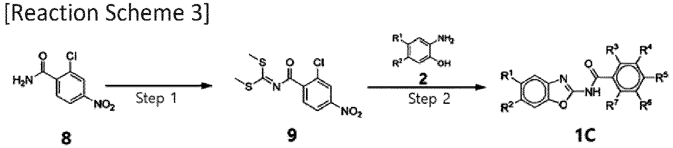

In Reaction Scheme 3,
R¹, R², R³, R⁴, R⁵, R⁶ and R⁷ are the same as defined in Chemical Formula 1 above, and
Compound 1C is included in Chemical Formula 1 above.

In the preparation method of the present invention, as an example of the organic solvent, methanol (MeOH), dimethylformamide (DMF), acetonitrile (MeCN), tetrahydrofuran (THF), dichloromethane (DCM), 1,2-dimethoxyethane, benzene, toluene, xylene, dimethyl sulfoxide (DMSO), or dioxane may be used alone or in combination.

In the preparation method of the present invention, an example of the compound that can be prepared by the above preparation method may be N-(benzo[d]oxazol-2-yl)-2-chloro-4-nitrobenzamide.

### Preparation method 4 of the compounds

The present invention provides a method for preparing a compound represented by Chemical Formula 1D, as shown in the following Reaction Scheme 4, the method including:
obtaining Compound 11 by dissolving Compound 10 and Compound 3 in an organic solvent, and then reacting the resulting solution at 10 to 50°C for 20 to 28 hours (Step 1);
obtaining Compound 12 by adding an organic solvent in which Compound 11 obtained in Step 1 is dissolved dropwise to an organic solvent in which potassium superoxide is dissolved, and then reacting the resulting mixture at 10 to 50°C for 12 to 24 hours (Step 2);
obtaining Compound 13 by adding Compound 12 together with a catalyst to an organic solvent, and then injecting hydrogen gas thereinto, and reacting the resulting mixture at 10 to 50°C for 12 to 20 hours (Step 3); and
obtaining Compound 1D by dissolving Compound 13 and Compound 14 in an organic solvent, and then reacting the resulting solution at 10 to 50°C for 12 to 24 hours (Step 4).

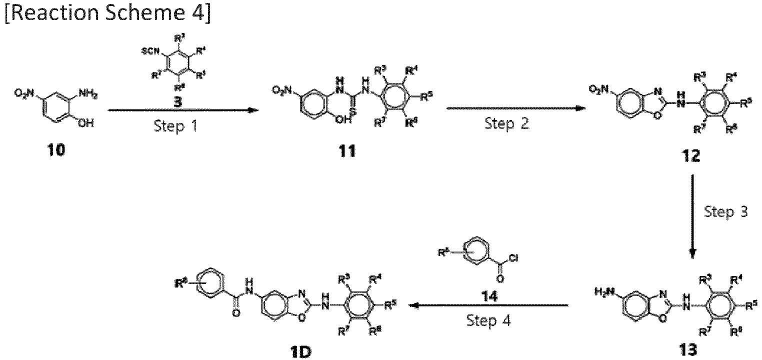

In Reaction Scheme 4,
R³, R⁴, R⁵, R⁶ and R⁷ are the same as defined in Chemical Formula 1 of claim 1;
R⁸ is one or more of a halogen, -NO₂ and a C₁₋₁₀ straight or branched alkyl halide; and
Compound 1D is included in Chemical Formula 1 of claim 1.

In the preparation method of the present invention, as an example of the organic solvent, methanol (MeOH), dimethylformamide (DMF), acetonitrile (MeCN), tetrahydrofuran (THF), dichloromethane (DCM), 1,2-dimethoxyethane, benzene, toluene, xylene, dimethyl sulfoxide (DMSO), or dioxane may be used alone or in combination.

In the preparation method of the present invention, an example of the compound that can be prepared by the above preparation method may be N-(2-(4-ethylphenylamino)benzo[d]oxazol-5-yl)-2-chloro-5-nitrobenzamide, N-(2-(4-ethylphenylamino)benzo[d]oxazol-5-yl)-3,4-dichlorobenzamide or N-(2-(4-ethylphenylamino)benzo [d]oxazol-5-yl)-3-(chloromethyl)benzamide.

In the present invention, the present composition can improve the expression level of Klotho gene.

In the present invention, the neurodegenerative disease comprises, but is not limited to, one or more diseases selected from the group consisting of stroke, amnesia, memory loss, memory impairment, dementia, forgetfulness, cognitive dysfunction, Parkinson's disease, Alzheimer's disease, Pick's disease, Creutzfeld-Kacob disease, Huntington's disease, and Lou Gehrig's disease.

The compound of the present invention may be administered in various oral and parenteral dosage forms, and during the formulation, the compound of the present invention is prepared using a diluent or an excipient, such as a filler, a thickener, a binder, a wetting agent, a disintegrant, and a surfactant which are commonly used.

A solid formulation for oral administration includes a tablet, a pill, a powder, a granule, a capsule, a troche, and the like, and the solid formulation is prepared by mixing at least one excipient, for example, a starch, calcium carbonate, sucrose or lactose, gelatin, and the like with one or more compounds of the present invention. Further, in addition to a simple excipient, lubricants such as magnesium stearate and talc are also used. A liquid preparation for oral administration corresponds to a suspension agent, a liquid for internal use, an emulsion, a syrup, and the like, and the liquid preparation may include various excipients, for example, a wetting agent, a sweetener, an aroma, a preservative, and the like, in addition to water and liquid paraffin which are commonly used simple diluents.

A preparation for parenteral administration includes an aqueous sterile solution, a non-aqueous solvent, a suspension solvent, an emulsion, a freeze-dried preparation, a suppository, or the like. As a non-aqueous solvent and a suspension solvent, it is possible to use propylene glycol, polyethylene glycol, a vegetable oil such as olive oil, an injectable ester such as ethyl oleate, and the like. As a base of the suppository, it is possible to use Witepsol, Macrogol, Tween 61, cacao butter, laurin fat, glycerol, gelatin, and the like.

In addition, the effective dosage of the compound of the present invention to the human body may vary depending on the patient's age, body weight, sex, administration form, health condition, and severity of disease, and is generally about 0.001 to 100 mg/kg/day, preferably 0.01 to 35 mg/kg/day. Based on an adult patient weighing 70 kg, the dosage is generally 0.07 to 7000 mg/day, preferably 0.7 to 2500 mg/day, and the compound of the present invention may be administered in divided doses once or several times a day at regular time intervals according to the judgment of a doctor or pharmacist.

### Food composition or health functional food composition for preventing or improving neurodegenerative disease

The present invention provides a food composition or a health functional food composition for preventing or improving neurodegenerative disease comprising the compound represented by Chemical Formula 1 or a pharmaceutically acceptable salt thereof.

In the present invention, the composition can improve the expression level of Klotho gene.

In the present invention, the neurodegenerative disease comprises, but is not limited to, one or more diseases selected from the group consisting of stroke, amnesia, memory loss, memory impairment, dementia, forgetfulness, cognitive dysfunction, Parkinson's disease, Alzheimer's disease, Pick's disease, Creutzfeld-Kacob disease, Huntington's disease, and Lou Gehrig's disease.

The type of food is not particularly limited. Examples of foods to which the active material of the present invention can be added include drinks, meats, sausages, bread, biscuits, rice cakes, chocolate, candies, snacks, confectioneries, pizza, instant noodles, other noodles, gums, dairy products including ice cream, various soups, drinking water, alcoholic beverages, vitamin complexes, milk products, dairy products, and the like, and include all health foods and health functional foods in a typical sense.

The health food and health functional food composition containing the active material according to the present invention may be added to food as it is or may be used together with other foods or food ingredients, and may be appropriately used according to a typical method. The mixing amount of the active material may be suitably determined depending on the purpose of use (for prevention or alleviation). In general, the amount of the composition in health foods and health functional foods may be 0.1 to 90 parts by weight of the total food weight. However, in the case of long-term intake for the purpose of maintaining health or for the purpose of health control, the amount may be equal to or less than the above range, and the effective material may be used in an amount equal to or more than the above range because it poses no problem in terms of safety.

Other ingredients are not particularly limited, other than that the health food and health functional food composition of the present invention contains the active material of the present invention as an essential ingredient at an indicated ratio, and the health food and health functional food composition of the present invention may contain various flavoring agents like those of a typical beverage, natural carbohydrates, and the like as additional ingredients. Examples of the above-described natural carbohydrates include typical sugars such as monosaccharides, for example, glucose, fructose and the like; disaccharides, for example, maltose, sucrose and the like; and polysaccharides, for example, dextrin, cyclodextrin and the like, and sugar alcohols such as xylitol, sorbitol, and erythritol. As flavoring agents in addition to those described above, a natural flavoring agent (thaumatin), a stevia extract (for example, rebaudioside A, glycyrrhizin and the like), and a synthetic flavoring agent (saccharin, aspartame and the like) may be advantageously used. The proportion of the natural carbohydrate is generally about 1 to 20 g, and preferably about 5 to 12 g per 100 g of the health functional food composition of the present invention.

The health food and health functional food composition containing the active material of the present invention may contain various nutrients, vitamins, minerals (electrolytes), flavoring agents such as synthetic flavoring agents and natural flavoring agents, colorants and thickening agents (cheese, chocolate, and the like), pectic acid and salts thereof, alginic acid and salts thereof, organic acids, protective colloid thickeners, pH adjusting agents, stabilizers, preservatives, glycerin, alcohols, carbonating agents used in carbonated beverages, or the like, in addition to the additives. In addition, the health food and health functional food composition of the present invention may contain flesh for preparing natural fruit juice, fruit juice drinks, and vegetable drinks.

These ingredients may be used either independently or in combination. The proportion of these additives is not particularly important, but is generally selected within a range of 0.1 to 20 parts by weight per 100 parts by weight of the health food and health functional food composition of the present invention containing the active material of the present invention.

Hereinafter, the present invention will be described in more detail with reference to the following examples. However, the following examples are only for exemplifying the present invention, and the content of the present invention is not limited by the following examples.

### <Example 1> Preparation of N-(benzo[d]oxazol-2-yl)-2-chloro-4-nitrobenzamide (FCCS-17064)

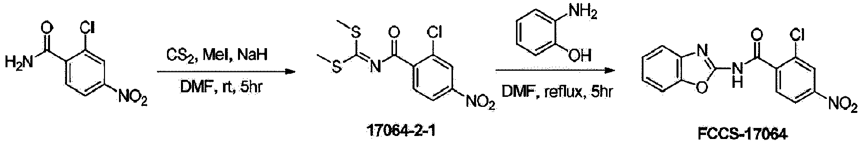

### Step 1: Preparation of dimethyl(2-chloro-4-nitrobenzoyl)carbonimidodithioate (17064-2-1)

After 2-chloro-4-nitrobenzamide (500 mg, 2.49 mmol), carbon disulfide (CS₂) (759 mg, 9.97 mmol), and iodomethane (1.13 g, 7.97 mmol) were dissolved in *N,N-*dimethylformamide (7 mL), 60% sodium hydride (200 mg, 4.98 mmol) was added thereto, and the resulting mixture was stirred at room temperature for 5 hours.

Ice-cold water was slowly added to the reaction mixture, and the mixture was extracted with ethyl acetate. The organic layer was washed with brine and dried over Na₂SO₄, and then the solvent was removed under reduced pressure. The reaction mixture was purified by silica-gel column chromatography (10% ethyl acetate/n-hexane) to obtain dimethyl(2-chloro-4-nitrobenzoyl)carbonimidodithioate (17064-2-1) as a light yellow solid (160 mg, 21%).

¹H NMR (400 MHz, acetone-d ₆); *δ* 8.34 (d, 1H, *J* = 2.0 Hz), 8.29 (dd, 1H, *J* = 2.4, 8.8 Hz), 8.20 (d, 1H, *J* = 8.4 Hz), 2.65 (s, 6H).

### Step 2: Preparation of N-(benzo[d]oxazol-2-yl)-2-chloro-4-nitrobenzamide (FCCS-17064)

After the dimethyl(2-chloro-4-nitrobenzoyl)carbonimidodithioate (17064-2-1) (150 mg, 0.49 mmol) obtained in Step 1 was dissolved in *N,N*-dimethylformamide (15 mL), 2-aminophenol (53 mg, 0.49 mmol) was added thereto.

After the reaction mixture was refluxed for 6 hours, the solvent was removed under reduced pressure. A solid obtained by adding diethyl ether to the reaction mixture and filtering a precipitated solid was purified by silica-gel column chromatography (40% ethyl acetate/n-hexane) to obtain a target compound N-(benzo[d]oxazol-2-yl)-2-chloro-4-nitrobenzamide (FCCS-17064) as a brown solid (70 mg, 30%).

¹H NMR (400 MHz, acetone-d₆); *δ* 8.36 (d, 1H, *J* = 2.0 Hz), 8.33 (dd, 1H, *J* = 2.0, 8.4 Hz), 8.09 (d, 1H, *J* = 8.4 Hz), 7.62-7.56 (m, 2H), 7.40-7.33 (m, 2H).

### <Example 2> Preparation of 8-methyl-2-[N-(3,4-dichlorophenyl)]aminobenzoxazole (FCCS-17065)

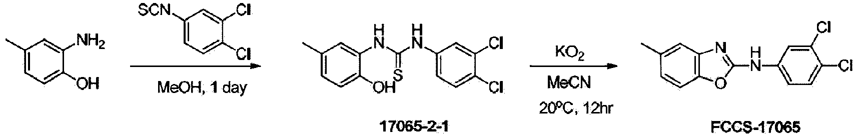

### Step 1: Preparation of 1-(3,4-dichlorophenyl)-3-(2-hydroxy-5-methylphenyl)thiourea (17065-2-1)

After 2-amino-*p*-cresol (300 mg, 2.44 mmol) was dissolved in methanol (12 mL), 3,4-dichlolrophenyl isothiocyanate (497 mg, 2.44 mmol) was added thereto, and the resulting mixture was stirred at room temperature for 18 hours. After the termination of the reaction was confirmed by thin layer chromatography (TLC), the resulting product was cooled in a refrigerator (0 to 4 °C). 1-(3,4-Dichlorophenyl)-3-(2-hydroxy-5-methylphenyl)thiourea (17065-2-1) as a white solid (346 mg) was obtained by filtering a precipitated solid, and was used in the next step without further purification.

¹H NMR (400 MHz, acetone-d₆); *δ* 7.98 (dd, 1H, *J* = 0.4, 2.0 Hz), 7.55-7.50 (m, 2H), 7.43 (br s, 1H), 6.94-6.90 (m, 1H), 6.85 (d, 1H, *J* = 8.4 Hz) 2.24 (s, 3H).

### Step 2: Preparation of 8-methyl-2-[N-(3,4-dichlorophenyl)]aminobenzoxazole (FCCS-17065)

After the 1-(3,4-dichlorophenyl)-3-(2-hydroxy-5-methylphenyl)thiourea (17065-2-1) (346 mg, 1.06 mmol) obtained in Step 1 in a solution of potassium superoxide (KO₂) (375 mg, 5.29 mmol) and acetonitrile (MeCN) (15 mL) was slowly added to a solution dissolved in acetonitrile (MeCN) (25 mL), the resulting mixture was stirred at room temperature for 18 hours.

Dichloromethane and water were added to the reaction mixture, and the mixture was extracted. The organic layer was washed with brine and dried over Na₂SO₄, and then the solvent was removed under reduced pressure. The reaction mixture was purified by silica-gel column chromatography (10% ethyl acetate/n-hexane to obtain a target compound 8-methyl-2-[N-(3,4-dichlorophenyl)]aminobenzoxazole (FCCS-17065) as a white solid (170 mg, 24%, 2 steps).

¹H NMR (400 MHz, acetone-d₆); *δ* 8.29 (d, 1H, *J* = 2.8 Hz), 7.73 (dd, 1H, *J* = 2.8, 8.8 Hz), 7.76 (d, 1H, *J* = 8.8 Hz), 7.32-7.20 (m, 1H), 7.28 (d, 1H, J = 8.0 Hz), 7.00-6.970 (m, 1H), 2.41 (s, 3H).

### <Example 3> Preparation of 2-((3,4-dichlorophenyl)amino)benzo[d]oxazol-5-ol (FCCS-17066)

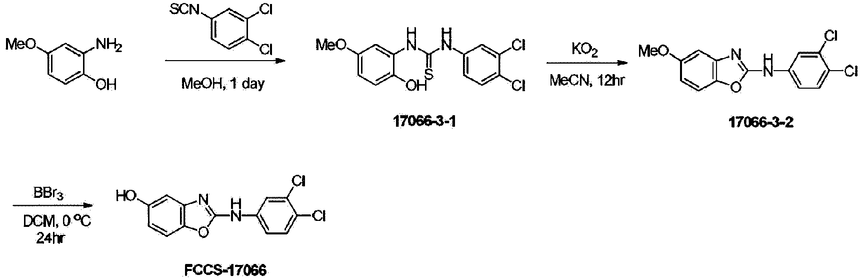

### Step 1: Preparation of 1-(3,4-dichlorophenyl)-3-(2-hydroxy-5-methoxyphenyl)thiourea (17066-3-1)

After 2-amino-4-methoxyphenol (1.13 g, 8.12 mmol) was dissolved in methanol (40 mL), 3,4-dichlolrophenyl isothiocyanate (1.99 g, 9.74 mmol) was added thereto, and the resulting mixture was stirred at room temperature for 18 hours. After the end of the reaction was confirmed by thin layer chromatography (TLC), the resulting product was cooled in a refrigerator (0 to 4 °C). 1-(3,4-Dichlorophenyl)-3-(2-hydroxy-5-methylphenyl)thiourea (17066-3-1) as a brown solid (2 g) was obtained by filtering a precipitated solid, and was used in the next step without further purification.

¹H NMR (400 MHz, methanol-d₄); *δ* 7.82 (d, 1H, *J* = 2.4 Hz), 7.48-7.44 (m, 2H), 7.39 (dd, 1H, *J* = 1.4, 8.8 Hz), 6.81 (d, 1H, *J* = 8.8 Hz), 6.66 (dd, 1H, *J* = 1.6, 8.8 Hz), 3.73 (s, 3H).

### Step 2: Preparation of N-(3,4-dichlorophenyl)-5-methoxybenzo[d]oxazol-2-amine (17066-3-2)

After the 17066-3-1 (525 mg, 1.52 mmol) obtained in Step 1 in a solution of potassium superoxide (KO₂) (540 mg, 7.6 mmol) and acetonitrile (MeCN) (20 mL) was slowly added to a solution dissolved in acetonitrile (MeCN) (30 mL), the resulting mixture was stirred at room temperature for 18 hours. Dichloromethane and water were added to the reaction mixture, and the mixture was extracted. The organic layer was washed with brine and dried over Na₂SO₄, and then the solvent was removed under reduced pressure. The reaction mixture was purified by silica-gel column chromatography (20% ethyl acetate/n-hexane) to obtain N-(3,4-dichlorophenyl)-5-methoxybenzo[d]oxazol-2-amine (17066-3-2) as a brown solid (230 mg, 35%).

¹H NMR (400 MHz, acetone-d₆); *δ* 8.29 (d, 1H, *J* = 2.4 Hz), 7.70 (dd, 1H, *J* = 2.4, 8.8 Hz), 7.55 (d, 1H, *J* = 8.8 Hz), 7.30 (d, 1H, *J* = 8.8 Hz), 7.08 (d, 1H, *J* = 2.8 Hz), 7.74 (dd, 1H, *J* = 2.4, 8.8 Hz), 3.84 (s, 3H).

### Step 3: Preparation of 2-((3,4-dichlorophenyl)amino)benzo[d]oxazol-5-ol (FCCS-17066)

After the 17066-3-2 (200 mg, 0.65 mmol) obtained in Step 2 was dissolved in dichloromethane (15 mL, anhydrous) in an Ar gas atmosphere, the resulting solution was cooled in an ice bath. Boron tribromide (BBr₃) (3.23 mL, 1.0 M in dichloromethane) was slowly added thereto, the temperature was increased to room temperature, and then the resulting mixture was stirred for 24 hours. The reaction was terminated by slowly adding a sodium hydroxide (NaOH) solution (8 mL, 1.0 M in water) thereto and the resulting product was transferred to a separatory funnel to separate an organic layer and an aqueous layer. The aqueous layer was extracted with ethyl acetate, and then dried over Na₂SO₄, and then the solvent was removed under reduced pressure. The reaction mixture was purified by silica-gel column chromatography (40% ethyl acetate/n-hexane) to obtain a target compound 2-((3,4-dichlorophenyl)amino)benzo[d]oxazol-5-ol (FCCS-17066) as a brown solid (97 mg, 50%).

¹H NMR (400 MHz, acetone-d₆); *δ* 8.29 (br s, -OH), 8.26 (d, 1H, *J* = 2.4 Hz), 7.72 (dd, 1H, *J* = 2.4, 8.8 Hz), 7.56 (d, 1H, *J* = 8.8 Hz), 7.21 (dd, 1H, *J* = 2.0, 7.2 Hz), 6.95 (d, 1H, *J* = 2.0 Hz), 6.66 (dd, 1H, *J* = 2.4, 8.8 Hz).

### <Example 4> Preparation of N-(2-(4-ethylphenylamino)benzo[d]oxazol-5-yl)-2-chloro-5-nitrobenzamide (FCCS-17067)

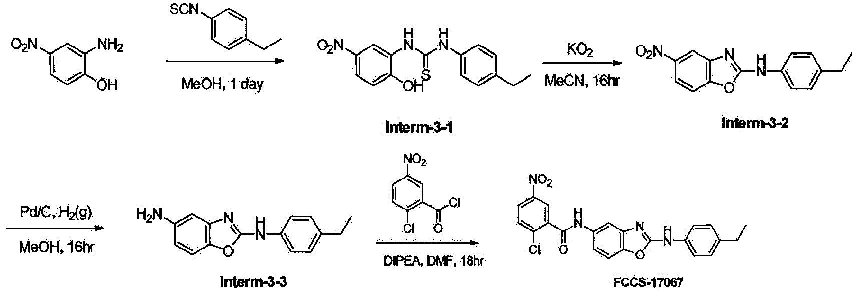

### Step 1: Preparation of 1-(4-ethylphenyl)-3-(2-hydroxy-5-nitrophenyl)thiourea (Interm-3-1)

After 2-amino-4-nitrophenol (1.88 g, 12.25 mmol) and 4-ethylphenyl isothiocyanate (2 g, 12.25 mmol) were dissolved in methanol (80 mL), the resulting solution was stirred at room temperature overnight. After the solvent was removed under reduced pressure, the residue was purified by silica-gel column chromatography (20% ethyl acetate/n-hexane) to obtain 1-(4-ethylphenyl)-3-(2-hydroxy-5-nitrophenyl)thiourea (Interm-3-1) as a brown solid (2.9 g, 65%).

¹H NMR (400 MHz, methanol-d₄); *δ* 9.24 (d, 1H, J = 2.8 Hz), 7.88 (dd, 1H, *J* = 2.0, 9.2 Hz), 7.36 (d, 2H, *J* = 8.4 Hz), 7.25 (d, 2H, *J* = 8.8 Hz), 6.94 (d, 1H, *J* = 9.2 Hz), 2.66 (q, 2H, *J* = 7.6 Hz), 1.24 (t, 3H, *J* = 7.6 Hz).

### Step 2: Preparation of N-(4-ethylphenyl)-5-nitrobenzo[d]oxazol-2-amine (Interm-3-2)

After a solution of potassium superoxide (I<Oz) (2.8 g, 39.38 mmol) and acetonitrile (MeCN) (130 mL) was cooled in an ice bath, a solution of the Interm-3-1 (2.5 g, 7.88 mmol) obtained in Step 1 dissolved in acetonitrile (MeCN) (170 mL) was slowly added thereto, and then the resulting solution was stirred at room temperature for 18 hours. Dichloromethane and water were added to the reaction mixture, and the mixture was extracted. The organic layer was washed with brine and dried over Na₂SO₄, and then the solvent was removed under reduced pressure. The reaction mixture was purified by silica-gel column chromatography (10% ethyl acetate/n-hexane) to obtain Compound Interm-3-2 as a brown solid (1.78 g, 80%).

¹H NMR (400 MHz, methanol-d₄); *δ* 8.20 (d, 1H, *J* = 1.0 Hz), 8.08 (dd, 1H, *J* = 0.8, 9.6 Hz), 7.59 (d, 2H, *J* = 8.8 Hz), 7.51 (d, 1H, *J* = 8.8 Hz), 7.22 (d, 2H, *J* = 8.8 Hz), 2.64 (q, 2H, *J* = 7.6 Hz), 1.24 (t, 3H, *J*= 7.6 Hz).

### Step 3: Preparation of N-(4-ethylphenyl)benzo[d]oxazole-2,5-diamine (Interm-3-3)

After palladium on carbon (Pd/C) (1.70 g, 0.80 mmol, 10 wt%, wet support) was weighed and put into a round-bottom flask, the flask was purged with Ar gas. After a solution of the Interm-3-2 (1.58 g, 5.30 mmol) obtained in Step 2 dissolved in methanol (80 mL) was slowly added thereto, the atmosphere in the flask was substituted with Hz(g). The solution was stirred at room temperature for 18 hours while bubbling H₂(g). After the termination of the reaction was confirmed by thin layer chromatography (TLC), the resulting product was filtered with a Celite pad, and then the solvent was removed under reduced pressure. The reaction mixture was purified by silica-gel column chromatography (40% ethyl acetate/n-hexane) to obtain Compound Interm-3-3 as a light brown solid (1.21 g, 90%).

¹H NMR (400 MHz, Acetone-d₆); *δ* 7.71 (m, 2 H), 7.19 (m, 2H), 7.03 (dd, 1H, *J* = 0.8, 8.4 Hz), 6.75 (dd, 1H, *J* = 0.8, 2.0 Hz), 6.44 (dd, 1H, *J* = 2.0, 8.4 Hz), 2.60 (q, 2H, *J* = 7.6 Hz), 1.19 (t, 3H, *J* = 7.6 Hz).

### Step 4: Preparation of N-(2-(4-ethylphenylamino)benzo[d]oxazol-5-yl)-2-chloro-5-nitrobenzamide (FCCS-17067)

After the Interm-3-3 (253 mg, 1 mmol) obtained in Step 3 and 2-chloro-5-nitrobenzoyl chloride (220 mg, 1 mmol) were dissolved in *N*,*N*-dimethylformamide (DMF) (4 mL), diisopropylethylamine (DIPEA) (129 mg, 1 mmol) was added thereto, and the resulting mixture was stirred at room temperature for 18 hours. After 18 hours, 0.5 equiv. of each of 2-chloro-5-nitrobenzoyl chloride and diisopropylethylamine (DIPEA) was added thereto, and the resulting mixture was further stirred for 8 hours. 10% HCl (aq.) was added to the reaction mixture, the mixture was extracted with ethyl acetate, and then the organic layer was sequentially washed with a saturated aqueous NaHCO₃ solution and brine. After the organic layer was dried over Na₂SO₄, the solvent was removed under reduced pressure. The reaction mixture was purified by silica-gel column chromatography (40% ethyl acetate/n-hexane) to obtain a target compound N-(2-(4-ethylphenylamino)benzo[d]oxazol-5-yl)-2-chloro-5-nitrobenzamide (FCCS-17067) as a light yellow solid (120 mg, 27%).

¹H NMR (400 MHz, DMSO-d₆); *δ* 10.71 (s, 1H), 10.53 (s, 1H), 8.48 (d, 1H, *J* = 2.8 Hz), 8.34 (dd, 1H, *J* = 2.4, 8.8 Hz), 7.90 (d, 1H, *J* = 8.8 Hz), 7.82 (d, 1H, *J* = 2.0 Hz), 7.64 (d, 2H, *J* = 8.8 Hz), 7.46 (d, 1H, *J* = 8.8 Hz), 7.40 (dd, 1H, J = 2.0, 8.4 Hz), 7.21 (d, 1H, *J* = 8.8 Hz), 2.58 (q, 2H, *J* = 7.6 Hz), 1.18 (t, 3H, *J* = 7.6 Hz).

### <Example 5> Preparation of N-(2-(4-ethylphenylamino)benzo[d]oxazol-5-yl)-3,4-dichlorobenzamide (FCCS-17068)

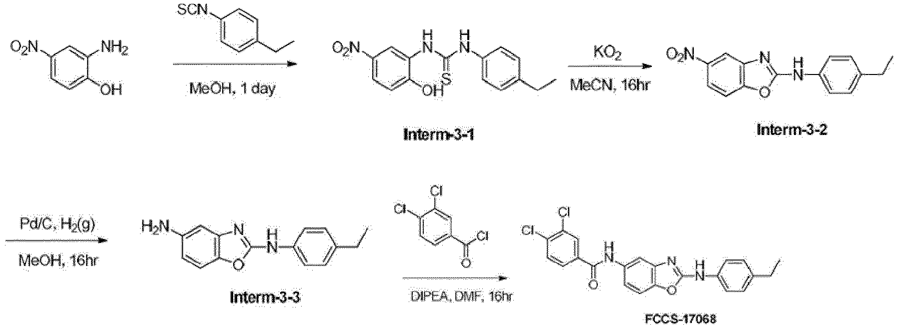

After the Interm-3-3 (253 mg, 1 mmol) obtained in Step 3 in Example 4 and 3,4-dichlorobenzoyl chloride (209 mg, 1 mmol) were dissolved in *N,N-*dimethylformamide (DMF) (4 mL), diisopropylethylamine (DIPEA) (129 mg, 1 mmol) was added thereto, and the resulting mixture was stirred at room temperature for 18 hours. 10% HCl (aq.) was added to the reaction mixture, the mixture was extracted with ethyl acetate, and then the organic layer was sequentially washed with a saturated aqueous NaHCO₃ solution and brine. After the organic layer was dried over Na₂SO₄, the solvent was removed under reduced pressure. The reaction mixture was purified by silica-gel column chromatography (40% ethyl acetate/n-hexane) to obtain a target compound FCCS-17067 as a light white solid (270 mg, 64%).

¹H NMR (400 MHz, Acetone-d₆); *δ* 8.18 (d, 1H, *J* = 2.4 Hz), 7.99 (t, 1H, *J* = 2.4 Hz), 7.97 (d, 1H, *J* = 2.0 Hz), 7.80-7.20 (m, 3H), 7.70-7.50 (m, 1H), 7.35 (d, 1H, *J* = 8.8 Hz), 7.24 (d, 1H, *J* = 8.8 Hz), 2.63 (q, 2H, *J* = 7.6 Hz), 1.22 (t, 3H, *J* = 7.6 Hz).

### <Example 6> Preparation of N-(2-(4-ethylphenylamino)benzo[d]oxazol-5-yl)-3-(chloromethyl)benzamide (FCCS-17069)

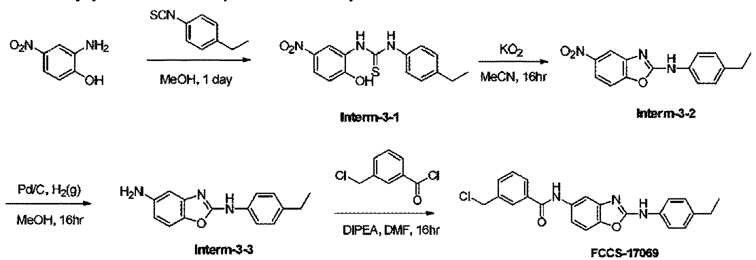

After the Interm-3-3 (253 mg, 1 mmol) obtained in Step 3 in Example 4 and 3-(chloromethyl)benzoyl chloride (189 mg, 1 mmol) were dissolved in *N,N-*dimethylformamide (DMF) (4 mL), diisopropylethylamine (DIPEA) (129 mg, 1 mmol) was added thereto, and the resulting mixture was stirred at room temperature for 18 hours. 10% HCl (aq.) was added to the reaction mixture, the mixture was extracted with ethyl acetate, and then the organic layer was sequentially washed with a saturated aqueous solution of NaHCO₃ and brine. After the organic layer was dried over Na₂SO₄, the solvent was removed under reduced pressure. The reaction mixture was purified by silica-gel column chromatography (30% ethyl acetate/n-hexane) to obtain a target compound FCCS-17067 as a pale white solid (170 mg, 40%).

¹H NMR (400 MHz, Acetone-d₆); *δ* 8.08 (t, 1H, *J* = 1.2 Hz), 8.03 (d, 1H, *J* = 2.0 Hz), 7.98 (dt, 1H, *J* = 1.2, 7.6 Hz), 7.80-7.75 (m, 2H), 7.69-7.65 (m, 1H), 7.57-7.52 (m, 3H), 7.34 (d, 1H, *J* = 8.8 Hz), 7.26-7.22 (m, 2H), 2.62 (q, 2H, *J* = 7.6 Hz), 1.22 (t, 3H, *J* = 7.6 Hz).

### <Example 7> Preparation of 2-[N-(3,4-dichlorophenyl)]aminobenzoxazole (FCCS-17065-A)

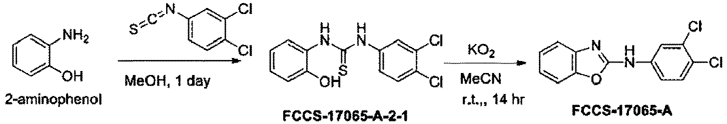

### Step 1: Preparation of 1-(3,4-dichlorophenyl)-3-(2-hydroxyphenyl)thiourea (FCCS-17065-A-2-1)

After methanol (anhydrous MeOH) (8 mL) was added to and dissolved in 2-aminophenol (300 mg, 2.749 mmol) in an Ar gas atmosphere, 3,4-dichlorophenyl isothiocyanate (0.47 mL, 3.299 mmol) was slowly added dropwise thereto and the resulting mixture was stirred at room temperature for 14 hours. It was confirmed by thin layer chromatography (TLC) that all starting materials had disappeared, the solvent was removed by reducing pressure, and then silica was added to and adsorbed to a crude product, and silica-gel flash column chromatography (30% EtOAc/hexane, R_{f}=0.4) was performed to obtain 793 mg (light brown foamy solid, 92%) of 1-(3,4-dichlorophenyl)-3-(2-hydroxyphenyl)thiourea (FCCS-17065-A-2-1).
¹H NMR (400 MHz, CD3OD); *δ* 7.82 (d, 1 H, *J* = 2.8 Hz), 7.63 (d, 1 H, J = 7.6 Hz), 7.45 (d, 1 H, *J* = 8.8 Hz), 7.39 (dd, 1 H, *J* = 8.6, 2.2 Hz), 7.11-7.06 (m, 1 H), 6.90 (dd, 1 H, *J* = 8.0, 1.2 Hz), 6.85 (td, 1 H, *J* = 7.6, 1.2 Hz)

### Step 2: Preparation of 2-[N-(3,4-dichlorophenyl)]aminobenzoxazole (FCCS-17065-A)

The FCCS-17065-A-2-1 (400 mg, 1.277 mmol) obtained in Step 1 and potassium superoxide (KO₂) (454 mg, 6.386 mmol) were put into a container in an Ar gas atmosphere, acetonitrile (MeCN) (48 mL) was added thereto, and the resulting mixture was stirred at room temperature for 14 hours. After it was confirmed by thin layer chromatography (TLC) that all starting materials had disappeared, silica was added to and adsorbed to a crude product crude, and pressure was reduced. Silica-gel flash column chromatography (20% EtOAc/hexane, R_{f}=0.4) was performed to obtain 231 mg (white solid, 65%) of a target compound 2-[N-(3,4-dichlorophenyl)]aminobenzoxazole (FCCS-17065-A).
¹H NMR (400 MHz, CD₃OD); *δ* 8.06 (d, 1 H, *J* = 2.8 Hz), 7.55 (dd, 1 H, *J* = 8.6, 2,6 Hz), 7.48-7.45 (m, 2 H), 7.39 (d, 1 H, *J* = 8.0 Hz), 7.24 (td, 1 H, *J* = 7.6, 1.2 Hz), 7.16 (td, 1 H, *J* = 7.8, 1.2 Hz)

### <Example 8> Preparation of N-(3,4-dichlorophenyl)naphtho[2,3-d]oxazol-2-amine (FCCS-17065-B)

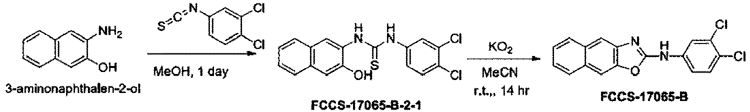

### Step 1: Preparation of 1-(3,4-dichlorophenyl)-3-(3-hydroxynaphthalen-2-yl)thiourea (FCCS-17065-B-2-1)

After methanol (anhydrous MeOH) (7 mL) and chloroform (CHCl₃) (2 mL) were added to 3-amino-2-naphthol (350 mg, 2.119 mmol) in an Ar gas atmosphere and the resulting mixture was stirred at room temperature for 5 minutes, 3,4-dichlorophenyl isothiocyanate (0.38 mL, 2.638 mmol) was slowly added dropwise thereto and the resulting mixture was stirred at room temperature for 13 hours. After it was confirmed by thin layer chromatography (TLC) that all starting materials had disappeared and the solvent was removed by reducing pressure, dichloromethane (8 mL) was added thereto, the resulting mixture was stirred for 5 minutes, and then a solid which had not been dissolved was filtered to obtain 792 mg (white solid, 99%) of 1-(3,4-dichlorophenyl)-3-(3-hydroxynaphthalen-2-yl)thiourea (FCCS-17065-B-2-1).
¹H NMR (400 MHz, DMSO-d₆); *δ* 10.48 (s, 1 H), 10.34 (s, 1 H), 9.57 (s, 1 H), 8.59 (s, 1 H), 8.05 (d, 1 H, *J* = 2.0 Hz), 7.73 (d, 1 H, *J* = 8.0 Hz), 7.67 (d, 1 H, *J* = 7.6 Hz), 7.60 (d, 1 H, *J* = 8.4 Hz), 7.52 (dd, 1 H, *J* = 8.6, 2.2 Hz), 7.35 (t, 1 H, *J* = 7.2 Hz), 7.27 (t, 1 H, *J* = 7.6 Hz), 7.24 (s, 1 H)

### Step 2: Preparation of N-(3,4-dichlorophenyl)naphtho[2,3-d]oxazol-2-amine (FCCS-17065-B)

The FCCS-17065-B-2-1 (400 mg, 1.101 mmol) obtained in Step 1 and potassium superoxide (KO₂) (391 mg, 5.505 mmol) were put into a container in an Ar gas atmosphere, acetonitrile (MeCN) (42 mL) was added thereto, and the resulting mixture was stirred at room temperature for 14 hours. After it was confirmed by thin layer chromatography (TLC) that all starting materials had disappeared, silica was added to and adsorbed to a crude product crude, and pressure was reduced. Silica-gel flash column chromatography (20% EtOAc/hexane, R_{f}=0.5) was performed to obtain 236 mg (white solid, 65%) of a target compound N-(3,4-dichlorophenyl)naphtho[2,3-d]oxazol-2-amine (FCCS-17065-B).
¹H NMR (400 MHz, DMSO-d₆); *δ* 11.22 (s, 1 H), 8.20 (d, 1 H, *J* = 2.0 Hz), 7.98-7.95 (m, 4 H), 7.72 (dd, 1 H, *J* = 8.8, 2.4 Hz), 7.66 (d, 1 H, *J* = 8.4 Hz), 7.47-7.41 (m, 2 H)

### <Example 9> Preparation of N-(3,4-difluorophenyl)-5-methylbenzo[d]oxazol-2-amine (FCCS-17065-C)

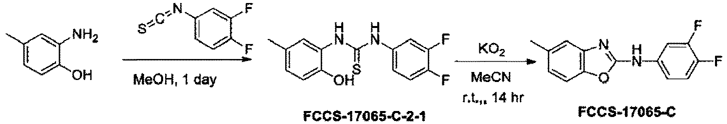

### Step 1: Preparation of 1-(3,4-difluorophenyl)-3-(2-hydroxy-5-methylphenyl)thiourea (FCCS-17065-C-2-1)

After methanol (anhydrous MeOH) (8 mL) was added to and dissolved in 2-amino-p-cresol (300 mg, 2.436 mmol) in an Ar gas atmosphere, 3,4-difluorophenyl isothiocyanate (0.37 mL, 2.923 mmol) was slowly added dropwise thereto and the resulting mixture was stirred at room temperature for 13 hours. It was confirmed by thin layer chromatography (TLC) that all starting materials had disappeared, the solvent was removed by reducing pressure, and then silica was added to and adsorbed to a crude product, and silica-gel flash column chromatography (30% EtOAc/hexane, R_{f}=0.4) was performed to obtain 710 mg (white foamy solid, 99%) of 1-(3,4-difluorophenyl)-3-(2-hydroxy-5-methylphenyl)thiourea (FCCS-17065-C-2-1).
¹H NMR (400 MHz, CD₃OD); *δ* 7.57-7.52 (m, 1 H), 7.40 (s, 1 H), 7.25-7.13 (m, 2 H), 6.91 (dd, 1 H, *J* = 8.0, 1.6 Hz), 6.79 (d, 1 H, *J* = 8.0 Hz), 2.25 (s, 3 H)

### Step 2: Preparation of N-(3,4-difluorophenyl)-5-methylbenzo[d]oxazol-2-amine (FCCS-17065-C)

The FCCS-17065-C-2-1 (400 mg, 1.359 mmol) obtained in Step 1 and potassium superoxide (KO₂) (483 mg, 6.795 mmol) were put into a container in an Ar gas atmosphere, acetonitrile (MeCN) (52 mL) was added thereto, and the resulting mixture was stirred at room temperature for 14 hours. After it was confirmed by thin layer chromatography (TLC) that all starting materials had disappeared, silica was added to and adsorbed to a crude product crude, and pressure was reduced. Silica-gel flash column chromatography (20% EtOAc/hexane, R_{f}=0.45) was performed to obtain 224 mg (white solid, 63%) of a target compound N-(3,4-difluorophenyl)-5-methylbenzo[d]oxazol-2-amine (FCCS-17065-C).
¹H NMR (400 MHz, CD₃OD); *δ* = 7.83-7.78 (m, 1 H), 7.33-7.29 (m, 1 H), 7.27-7.20 (m, 3 H), 6.97-6.95 (m, 1 H), 2.41 (s, 3 H)

### <Example 10> Synthesis of N-(3,4-difluorophenyl)benzo[d]oxazol-2-amine (FCCS-19025)

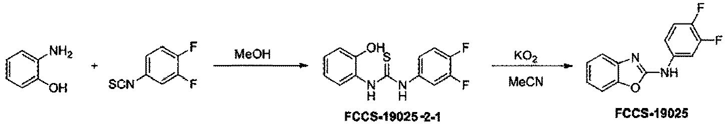

### Step 1: Preparation of 1-(3,4-difluorophenyl)-3-(2-hydroxyphenyl)thiourea (FCCS-19025-2-1)

After 2-aminophenol (150 mg, 1.37 mmol) was dissolved in methanol (8 mL) in an Ar gas atmosphere, 3,4-difluorophenyl isothiocyanate (224 µl, 1.65 mmol) was slowly added thereto, and then the resulting mixture was stirred at room temperature for 13 hours. After the termination of the reaction was confirmed by thin layer chromatography (TLC), methanol was removed under reduced pressure. The reaction mixture was purified by silica-gel column chromatography (20% acetone/n-hexane) to obtain 1-(3,4-difluorophenyl)-3-(2-hydroxyphenyl)thiourea (FCCS-19025-2-1) as a light yellow solid (354 mg, 92%).

¹H-NMR (400 MHz, MeOH-d₄) *δ* 7.62 (d, *J* = 8.0 Hz, 1H), 7.55 (ddd, *J*= 2.4 Hz, 1H), 7.25-7.13 (m, 2H), 7.11-7.05 (m, 1H), 6.92-6.82 (m, 2H); ESI-(+) 281.3 [M+H]⁺.

### Step 2: Preparation of N-(3,4-difluorophenyl)benzo[d]oxazol-2-amine (FCCS-19025)

The FCCS-19025-2-1 (224 mg, 0.80 mmol) obtained in Step 1 and potassium superoxide (KO₂) (284 mg, 4.00 mmol) were dissolved in acetonitrile (MeCN) (25 mL) in an Ar gas atmosphere, the resulting mixture was stirred at room temperature for 14 hours. After the termination of the reaction was confirmed by thin layer chromatography (TLC), acetonitrile (MeCN) was removed under reduced pressure. The reaction mixture was purified by silica-gel column chromatography (10 to 20% ethyl acetate/n-hexane) to obtain a target compound N-(3,4-difluorophenyl)benzo[d]oxazol-2-amine (FCCS-19025) as a white solid (160 mg, 82%).

¹H-NMR (400 MHz, MeOH-d₄) *δ* 7.82 (ddd, J= 2.8 Hz, 1H), 7.42 (d, *J* = 7.6 Hz, 1H), 7.36 (d, *J* = 8.0 Hz, 1H), 7.34-7.29 (m, 1H), 7.28-7.18 (m, 2H), 7.16-7.10 (m, 1H); ESI-(+) 247.2 [M+H]⁺.

The chemical structural formulae of Examples 1 to 10 are shown in the following Table 1.

**[Table 1]**

| Example | Chemical structural formula | Example | Chemical structural Formula |
|---|---|---|---|
| Example 1 (FCCS-17064) | 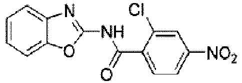 | Example 6 (FCCS-17069) | 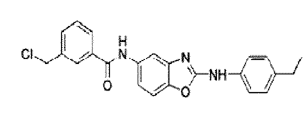 |
| Example 2 (FCCS-17065) | 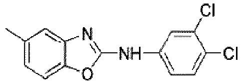 | Example 7 (FCCS-17065-A) | 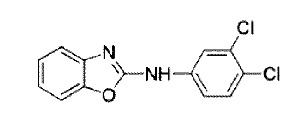 |
| Example 3 (FCCS-17066) | 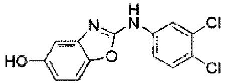 | Example 8 (FCCS-17065-8) | 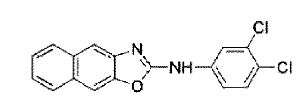 |
| Example 4 (FCCS-17067) | 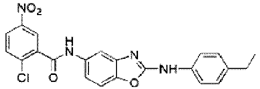 | Example 9 (FCCS-17065-C) | 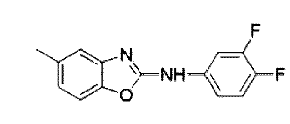 |
| Example 5 (FCCS-17068) | 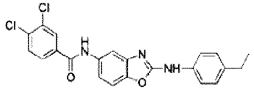 | Example 10 (FCCS-19025) | 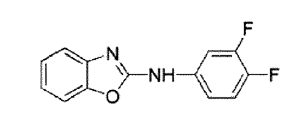 |

### <Comparative Example 1>

N-(2-chlorophenyl)-1H-indole-3-carboxamide was used as Comparative Example 1.

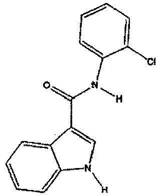

### <Comparative Example 2>

2'-Chloroacetanilide (C0621) was purchased and used as Comparative Example 2.

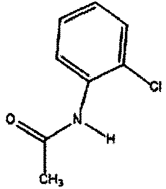

### <Comparative Example 3>

N-methyl-1H-indole-3-carboxamide (FCCS-16030) was purchased and used as Comparative Example 3.

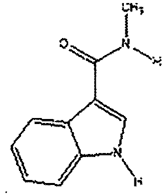

### <Comparative Example 4> Preparation of N-(2-((2-chlorophenyl)amino)-2-oxoethyl)-1H-indole-3-carboxamide (FCCS-16031)

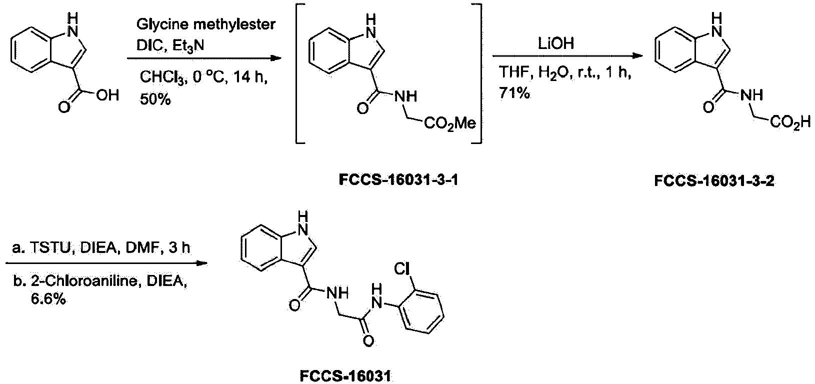

### Step 1: Preparation of methyl 2-(1H-indole-3-carboxamido)acetate (CCS-16031-3-1)

Indole-3-carboxylic acid (600 mg, 3.72 mmol) and glycine methyl ester (467 mg, 3.72 mmol) were dissolved in chloroform (11 mL), and the resulting solution was cooled in an ice bath. After triethylamine (1.04 mL, 7.446 mmol) and N,N-diisopropylcarbodiimide were additionally added thereto, the resulting mixture was stirred at 0°C for 14 hours. The mixture was washed with a 10% aqueous NaHCO₃ solution, and then washed with a 5% HCl aqueous solution, and the remaining moisture was removed by allowing the resulting mixture to pass through an anhydrous Na₂SO₄ pad, and then the solvent was removed under reduced pressure. 430 mg (white solid, 50%) of methyl 2-(1H-indole-3-carboxamido)acetate (CCS-16031-3-1) was obtained in a mixture state by performing silica-gel flash column chromatography (70% ethyl acetate/n-hexane). The next step was performed without further purification. ESI-MS : 231.2 [M-H]⁻

### Step 2: Preparation of 2-(1H-indole-3-carboxamido)acetic acid (FCCS-16031-3-2)

The methyl 2-(1H-indole-3-carboxamido)acetate (CCS-16031-3-1) (220 mg, 0.947 mmol) was dissolved in tetrahydrofuran (6 mL), a solution of a lithium hydroxide hydrate (LiOH monohydrate) (131 mg, 3.126 mmol) dissolved in water (2 mL) was added thereto, and the resulting mixture was stirred for 1 hour. After the pH was adjusted to 2 by adding a 1.0 N aqueous HCl solution, the mixture was extracted with ethyl acetate. After the remaining moisture was removed by allowing the mixture to pass through an anhydrous Na₂SO₄ pad, the solvent was removed under reduced pressure. 147 mg (yellow foamy solid, 71%) of 2-(1H-indole-3-carboxamido)acetic acid (FCCS-16031-3-2) was obtained by performing silica-gel flash column chromatography (10% methanol/dichloromethane).
¹H NMR (400 MHz, CD₃OD); *δ* 8.10-8.08 (m, 1 H), 7.92 (s, 1 H), 7.43 (dt, *J* = 8.0, 1.2 Hz, 1 H), 7.17 (quint d, *J* = 7.2,1.6 Hz, 2 H), 4.12 (s, 2 H)

### Step 3: Preparation of N-(2-((2-chlorophenyl)amino)-2-oxoethyl)-1H-indole-3-carboxamide (FCCS-16031)

The 2-(1H-indole-3-carboxamido)acetic acid (FCCS-16031-3-2) (200 mg, 0.917 mmol) and N,N,N',N'-tetramethyl-O-(N-succinimidyl)uronium tetrafluoroborate (TSTU) (290 mg, 0.962 mmol) were dissolved in N,N-dimethylformamide (4 mL, anhydrous) in an Ar gas atmosphere, N,N-diisopropylethylamine (DIEA) (0.4 mL, 2.293 mmol) was added thereto, and then the resulting mixture was stirred at room temperature for 3 hours. 2-Chloroaniline (0.29 mL, 2.751 mmol) and N,N-diisopropylethylamine (DIEA) (0.64 mL, 3.668 mmol) were added thereto, and the resulting mixture was heated at 60°C for 4 hours. After the solvent was removed under reduced pressure, an organic layer was obtained by adding dichloromethane and a saturated aqueous NH₄Cl solution thereto for extraction, the remaining moisture was removed by allowing the organic layer to pass through an anhydrous Na₂SO₄ pad, and then the solvent was removed under reduced pressure. 20 mg (white solid, 6.6%) of a target compound N-(2-((2-chlorophenyl)amino)-2-oxoethyl)-1H-indole-3-carboxamide (FCCS-16031) was obtained by performing silica-gel flash column chromatography (70% ethyl acetate/n-hexane).
1H NMR (400 MHz, CD₃OD); *δ* 8.15-8.12 (m, 1 H), 8.04 (dd, *J* = 8.0, 1.2 Hz, 1 H), 7.97 (s, 1 H), 7.46-7.41 (m, 2 H), 7.33-7.28 (m, 1 H), 7.23-7.12 (m, 3 H), 4.26 (s, 2 H)

### <Experimental Example 1-1> Luciferase expression experiment (Comparative Examples 1 to 4)

In order to evaluate whether the klotho gene was expressed by the evaluation of luciferase activity, human renal proximal tubule epithelial cells (RPTECs) (ATCC CRL-4031), which are epithelial cells of the proximal tubule of the human kidneys, were purchased from Lonza, USA and used.

For culturing, a Renal Epithelial Growth Medium (REGM^{™}) Bullet kit, also manufactured by the same Lonza company, was used, and the cells were cultured under conditions of 37 °C and 5% CO₂. As a plasmid for luciferase expression, a plasmid in which the promoter site of a human KL (klotho) gene was disposed to regulate the expression of a firefly luciferase gene was used.

The plasmid was incorporated into cells using an X-treme GENE transfection reagent from Roche. The activity of luciferase expressed in cells was measured using a Dual-Luciferase reporter assay system manufactured by Promega. After the cultured cells were treated with each compound at an indicated concentration for 24 hours, the activity of luciferase was measured. It is indirectly shown that the expression of the klotho gene may be increased when the activity of luciferase is high.

The expression of a reporter gene was confirmed by treating RPTEC cells with Comparative Examples 1 to 4 at a concentration of 5 µM and using a reporter gene including a promoter from the start site of the human klotho gene to a front of 1.7 kbp or a reporter gene including a promoter from the start site of the human klotho gene to a front of 240 bp.

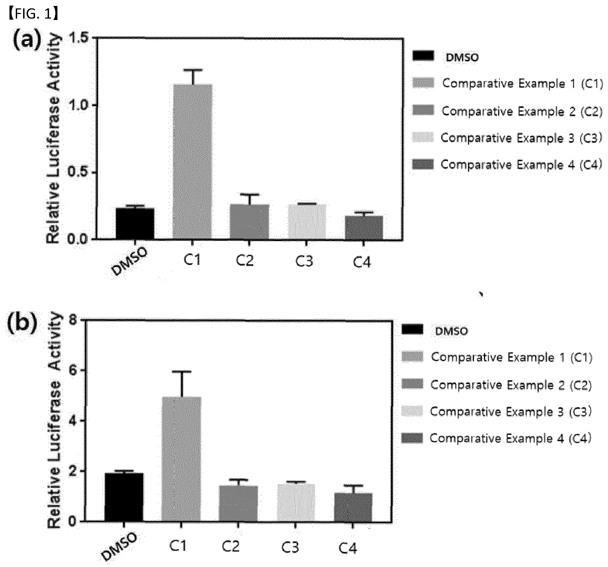

As a result, as illustrated in FIG. 1, it was confirmed that the luciferase activity of the compound of Comparative Example 1 was the highest.

### <Experimental Example 1-2> Luciferase expression experiment (Examples 1 to 6)

Based on the results of Experimental Example 1-1, Examples 1 to 6 having a chemical structure similar to that of Comparative Example 1 were synthesized, and the present Experimental Example 1-2 was performed.

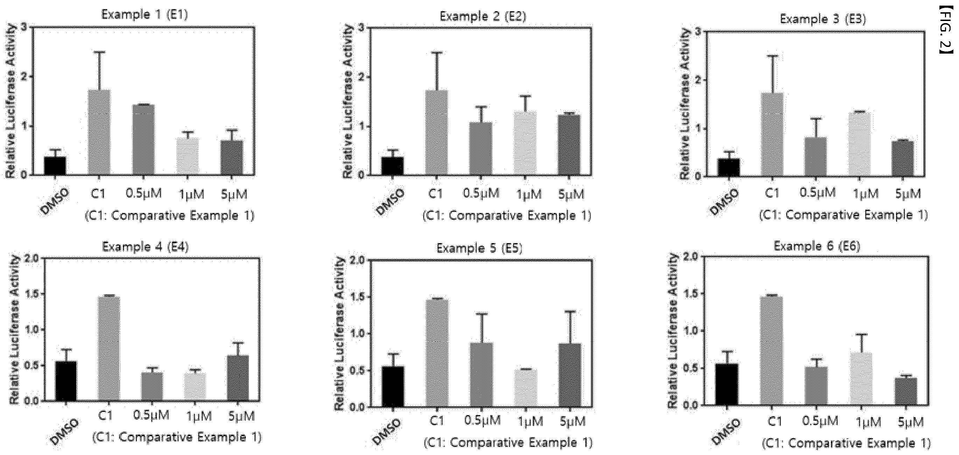

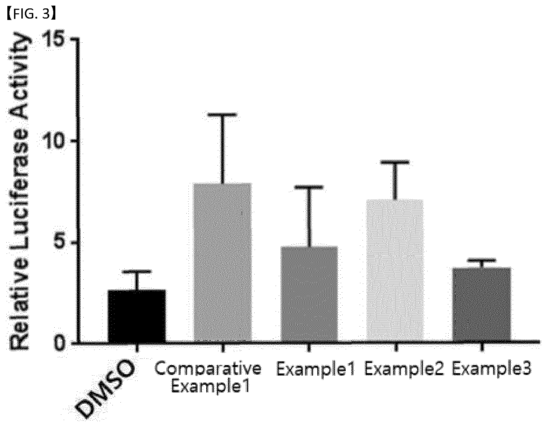

The results of confirming the expression of a reporter gene by treating RPTECs, which are epithelial cells of the proximal tubule of the human kidneys, with Examples 1 to 6 at concentrations of 0.5, 1 and 5 µM and Comparative Example 1 at a concentration of 5 µM and using a reporter gene (pHKP-luc) including a promoter included up to -2.1 kb upstream of the human klotho gene are illustrated in FIGS. 2 and 3.

As illustrated in FIG. 2, it was confirmed that the expression of the reporter genes of the compounds of Examples 1 and 2 was at a level similar to that of Comparative Example 1.

As illustrated in FIG. 3, as a result of confirming the expression of a reporter gene by treating RPTEC cells with Comparative Example 1 and Examples 1 to 3 at a concentration of 5 µM and using the reporter gene (pHKP-luc) including a promoter included up to -2.1 kb upstream of the human klotho gene, it was confirmed that the compound of Example 2 was at a level similar to that of Comparative Example 1.

### <Experimental Example 2> Quantitative evaluation of klotho (KL) gene expression level using real-time PCR experiment

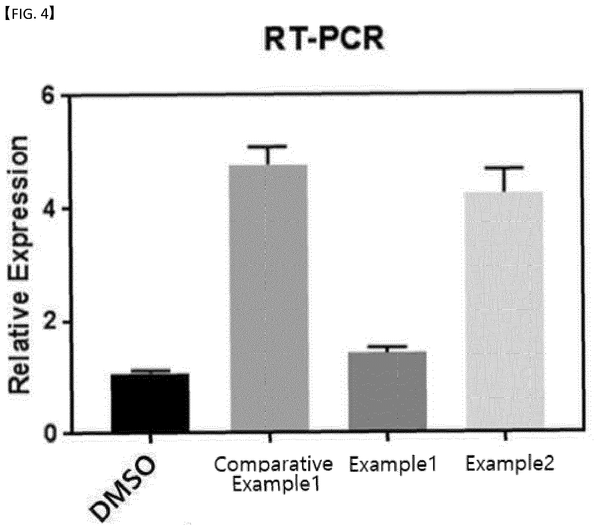

For RNA extraction from RPTEC cells, which are epithelial cells of the proximal tubule of the human kidneys, treated with the compounds of Comparative Example 1 and Examples 1 and 2 for 6 hours, an RNeasy kit from Qiagen Inc. was used. The results of preparing cDNA from extracted RNA using a Superscript II kit from Thermo Fisher Scientific, Inc. and performing the quantitative evaluation using Taqman Gene Expression assays from Applied Biosystems as a klotho (KL) gene-specific kit are illustrated in FIG. 4.

As illustrated in FIG. 4, it was confirmed that the compound of Example 2 was at a level similar to that of Comparative Example 1.

Based on the results of the present Experimental Example 2, the compounds of Examples 7 to 10 having a chemical structure similar to the compound of Example 2 were synthesized and used in the following Experimental Example 3.

### <Experimental Example 3> Quantitative evaluation of klotho (KL) gene expression level using general PCR experiment

After RPTEC cells, which are epithelial cells of the proximal tubule of the human kidneys, were each treated with the compounds of Examples 7 to 10 at 2.5 µM for 6 hours, RNA was extracted from the cells, and cDNA was prepared from the extracted RNA using a Superscript II kit from Thermo Fisher Scientific, Inc., and then general PCR was performed.

Information on the primers used in the experiment is as follows.
KL-F GATAGAGAAAAATGGCTTCCCTCC (SEQ ID NO: 1)
KL-R GGTCGGTAAACTGAGACAGAGTGG (SEQ ID NO: 2)
GAPDH-F TGACAACTTTGGTATCGTGGAAGG (SEQ ID NO: 3)
GAPDH-R AGGGATGATGTTCTGGAGAGCC (SEQ ID NO: 4)

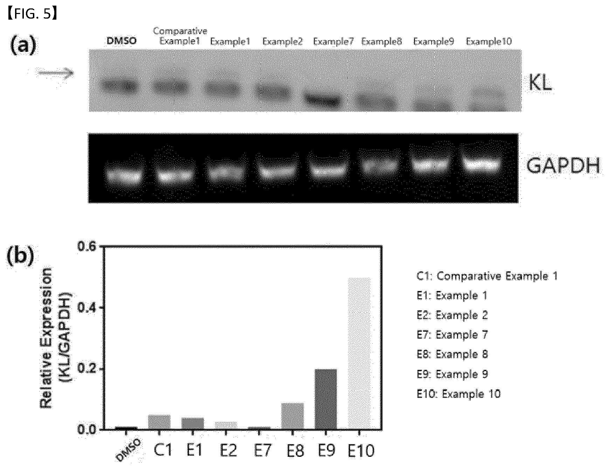

The DNA amplified by PCR was confirmed by staining with ethidium bromide after electrophoresis on an agarose gel, and the amounts of DNA in the band were quantitatively compared using the SPEedyQuant program, and are illustrated in FIG. 5.

As illustrated in FIG. 5, it was confirmed that the expression level of the klotho (KL) gene in Examples 8 to 10 was much higher than that in Comparative Example 1, and in particular, Example 10 showed an improvement to about 10 times the level of Comparative Example 1.

### <Experimental Example 4> Toxicity test

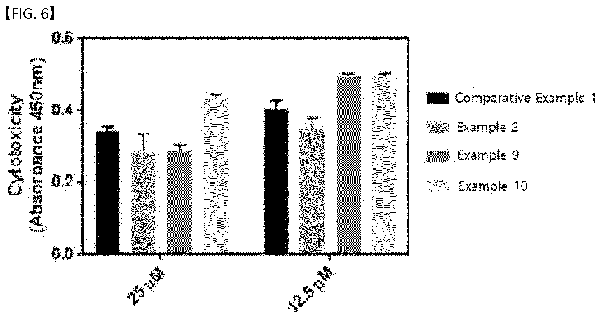

Cultured human kidney-2 (HK2) cells were treated with Comparative Example 1, Example 2 and Examples 9 to 10 at a concentration of 25 µM or 12.5 µM, and after 24 hours, cytotoxicity was measured using an EZ-Cytox kit. Since EZ-Cytox produces formazan having an absorbance at 450 nm by mitochondrial enzymes in living cells, living cells exhibit even higher absorbance at 450 nm. When the toxicity of cells treated with DMSO in the same volume as the amount of treated compound was set to 1, it was confirmed how much the cells were toxically reduced by the compound sample treatment, and the results are illustrated in FIG. 6.

As illustrated in FIG. 6, it was confirmed that when the cells were treated at a concentration of 12.5 µM or 25 µM, the compound of Example 10 showed the least toxicity, and the toxicity showed an improvement of 20% or more even when compared to Comparative Example 1.

### <Experimental Example 5 > Analysis of effect of neuronal senescence inhibition by KS 1 compound (Example 10)

### 1) Cell culture and treatment of compound

HT22 cells, neuronal cells derived from mouse hippocampus, were cultured in DMEM culture medium supplemented with 10% FBS. Cell cultures were incubated in a 37°C incubator at 5% CO₂, and when the cells grew to saturate the surface of the culture dish by 80%, they were treated with 0.05% trypsin and transferred to new culture dishes for passage. The compound was added to the medium at each passage to maintain a final concentration of 2.5 µM. Cells with only DMSO as solvent were used as a negative control, and cells from three passages (#5) were used as a non-aging control. All senescent cells were cultured in 20 passages (#25).

### 2) Checking the degree of senescence

To check the degree of senescence of the cells, "Senescence β-galactosidase staining kit" from Cell Signaling Technology was used. The staining solution was added to the cells on the day of the experiment and the stained cells were checked 8 hours later. The number of stained cells was determined and expressed as a percentage of the total number of cells observed under a 200x microscope, and the number of stained cells in three different locations per sample was counted and averaged. As the expression of β-galactosidase enzyme increases as the aging of cells progresses, stained cells can be judged as more aged cells than unstained cells.

### 3) Experimental results

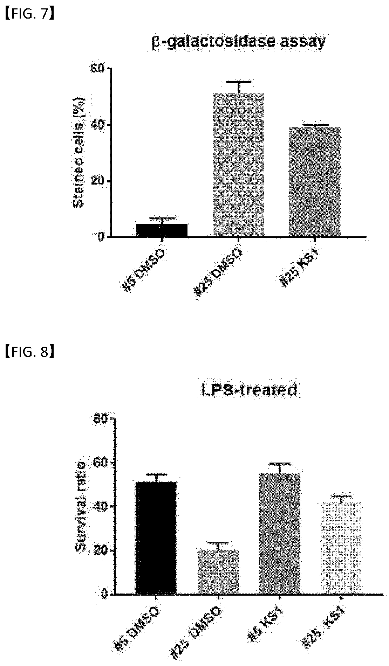

The experimental results showed that cells cultured for 20 passages showed an increased degree of senescence compared to cells cultured for 5 passages. When observed under a microscope at 200x, about 50% of the cells in the 20 passages were stained, while only 5% of the cells in the 5 passages were stained. In a group of cells cultured for the same 20 passages but with KS1 compound (Example 10) in the medium, the degree of staining decreased to 40% of the cells. In other words, cells cultured in the presence of KS1 compound (Example 10) showed a 20% decrease in the degree of senescence compared to cells grown in medium containing DMSO during the 20 passages (FIG. 7).

### <Experimental Example 6 > Analysis of effect of inhibiting neuronal inflammation by KS 1 compound (Example 10)

### 1) Cell culture and treatment of compound

HT22 cells, neuronal cells derived from mouse hippocampus, were cultured in DMEM culture medium supplemented with 10% FBS. Cell cultures were incubated in a 37°C incubator at 5% CO₂, and when the cells grew to saturate the surface of the culture dish by 80%, they were treated with 0.05% trypsin and transferred to new culture dishes for passage. The compound was added to the medium at each passage to maintain a final concentration of 2.5 µM. Cells with only DMSO as solvent were used as a negative control, and cells from three passages (#5) were used as a non-aging control. All senescent cells were cultured in 20 passages (#25).

### 2) Inflammation induction experiments

Cells were seeded at 10,000 cells in 160 µl of medium into each well of a 96-well plate and incubated in a 37°C incubator at 5% CO² for 24 h. The compounds were added to the culture medium to a final concentration of 2.5 µM. After incubation, LPS (lipopolysaccharide) from Cell application was added at a concentration of 1 µg/mL and incubated for another 24 hours. The Cyto X cell viability assay kit from the same company was then added 20 µl to each well and incubated for 1 to 4 hours. The color change was then measured at 450 nm.

### 3) Experimental results

The results showed that cells cultured in 20 passages showed increased toxicity of LPS treatment compared to cells cultured in 5 passages. When the change in absorbance was measured by an absorbance spectrophotometer, about 80% of the cells cultured in 20 passages with DMSO were killed by LPS toxicity, but only 50% of the cells cultured in 5 passages were killed, indicating that relatively more cell death occurred in the aged cells. For cells cultured in medium supplemented with KS1 compound (Example 10), the percentage of cells killed by LPS treatment was 60% in cells cultured for 20 passages, while 45% of cells were killed in cells cultured for 5 passages. In other words, there was a reduction in LPS-induced cytotoxicity in cells cultured in the presence of KS1 compound (Example 10), indicating that more cells were found to be alive, indicating that KS1 compound (Example 10) is effective in inhibiting inflammation in neurons (FIG. 8).

### <Experimental Example 7> Experiment to confirm the therapeutic effect of the KS1 compound in cognitive dysfunction animal models

### 1) Animal model and administration method of KS1 compound (Example 10)

In this experiment, 5xFAD mice aged 4 months or 6 months were used. 5xFAD mice are Alzheimer's mice genetically modified to overproduce beta amyloid (Aβ, β amyloid) and were provided by the KIST Research Animal Resource Center, which maintains and sells them. Normal control (WT) mice, which are not genetically modified to overproduce β-amyloid (Aβ), were provided by the KIST Research Animal Resource Center. The animals were acclimatized for 1 week before the experiments. Mice were kept on an ad libitum diet in an animal room maintained at 22 ± 2 °C and 40-60% humidity, and the light and dark cycles were controlled at 12-hour intervals. All animal experiments were performed in accordance with the animal care and use regulations of the Institutional Animal Care and Use Committee of the Korea Advanced Institute of Science and Technology (KIST).

Animal model mice and normal control mice received vehicle (5% DMSO + 65% PEG400 + 30% Saline) or vehicle with KS1 (Example 10) dissolved in it orally (p.o.) at a concentration of 10 mg/kg daily for 4 or 12 weeks. Three mice were used per experimental group. The body weight was measured weekly during the treatment period, and after the end of the treatment, the cognitive function was tested and sacrificed, and the organs were harvested to confirm the changes through biochemical methods.

### 2) Test methods to check cognitive function

### 2-1) Novel object recognition test (NOR)

The novel object recognition test is a modification of the existing method and consists of an adaptation period, an exploration period, and a novel object recognition period. The day before the test, the mice were placed in an open field for 30 minutes to acclimatize, and then two identical objects were placed at regular intervals. The animals were allowed to explore freely for 10 minutes and the time spent on each object was measured using Ethovision before being returned to the cage for one day. The next day, one of the objects was replaced with a new object and the animal's movement was measured for 10 minutes. The recognition index (%) was calculated as the time spent on each object as a percentage of the total time spent exploring.

### 2-2) Passive avoidance experiment

The passive avoidance apparatus was divided into two sections, one with light and the other with darkness, and the floor was made of wire mesh. During training, the animals were placed in the light area, and as soon as they moved into the dark area, the door was closed and they received a foot shock with a current of 0.45 mA for 2 seconds. The test was performed one day after the training, and the time from when the animals were placed in the bright area to moving back to the dark area was measured (the maximum time was set at 600 seconds). This passive avoidance test was used to assess spatial learning and memory.

### 2-3) Statistical processing

All data were expressed as mean (S.E.M), and differences between groups were determined using one-way ANOVA and Tukey's multiple comparisons test. When it was determined that there was a statistical difference, p<0.05 *, p<0.01 **, p<0.001 *** were indicated.

### 3) Experimental Results

### 3-1) Reduced expression of cognitive dysfunction proteins

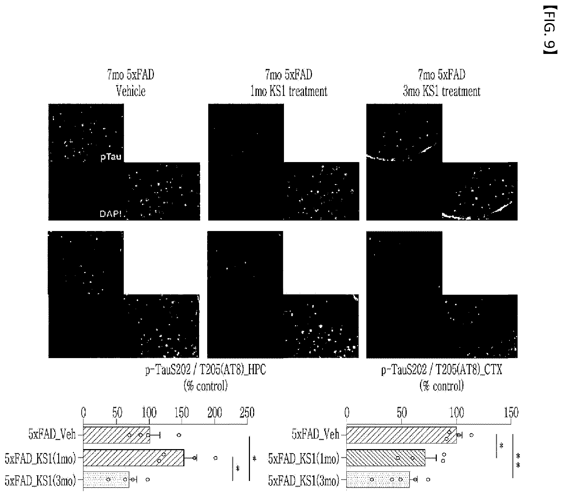

The fluorescence levels of phospho-Tau measured in each region (HPC: hippocampus; CTX: neocortex) of brain tissue sections isolated from control (CTL) and KS11-month and 3-month treatment groups of 5xFAD cognitive dysfunction animal model and normal control animal model were investigated by Immunohistochemistry. The results showed that the expression of phospho-Tau protein was reduced to a statistically significant level in mice treated with KS1 (Example 10) compared to mice treated with vehicle. In the hippocampus (HPC) region, reduced expression of the proteins was seen in only the 3-month treated mice, and in the primary somatosensory cortex (CTX) region, reduced expression of the proteins was seen in both 1- and 3-month treated mice (FIG. 9).

### 3-2) Increased expression of neuronal marker NeuN

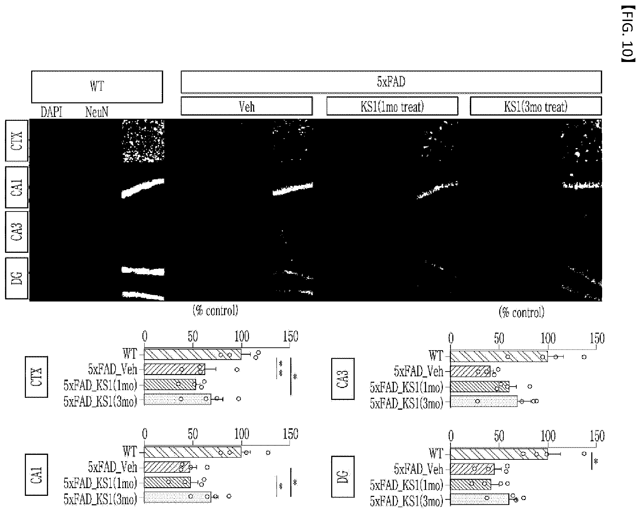

The fluorescence level of NeuN measured in each region (CTX: neocortex, CA: amygdala, DG: dentate gyrus) of brain tissue sections isolated from control (CTL) and KS11-month and 3-month treatment groups of 5xFAD cognitive dysfunction animal model and normal control animal model was investigated by Immunohistochemistry. The results showed that the expression of NeuN neuromarker protein was increased to a statistically significant level in mice treated with KS1 (Example 10) compared to mice treated with vehicle (FIG. 10). In the primary somatosensory cortex (CTX) and dentate gyrus (DG), no significant increase in NeuN by KS1 could be confirmed, but in the Cornu Ammonis (CA), an increase in NeuN was confirmed in the KS1-treated group.

### 3-3) Novel Object Recognition Test (NOR)

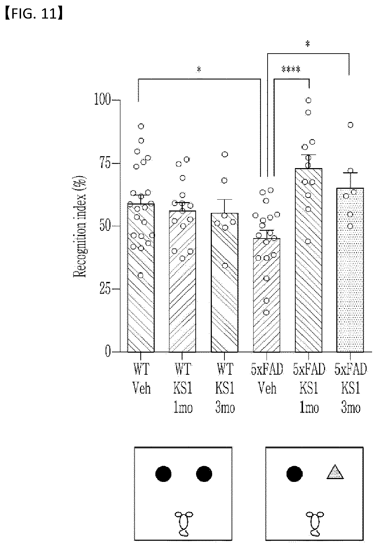

When exploring the behavior of mice using the NOR method, we found that the cognitive dysfunction (5xFAD) mice treated with KS1 showed a statistically significant increase in object detection ability compared to mice treated with vehicle (Fig. 11).

### 3-4) Passive Avoidance Experiment

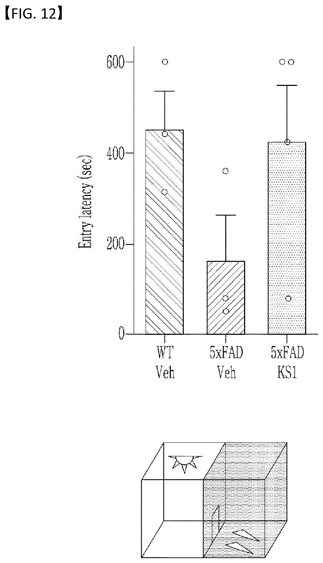

As a result of testing spatial learning and memory through the passive avoidance test, we found that the cognitive dysfunction (5xFAD) mice showed a significant decrease in performance compared to normal mice, but the mice treated with KS1 for 4 weeks maintained a similar level of performance to normal mice (Fig. 12).

So far, the present invention has been looked at with respect to its preferred embodiments. Those skilled in the art to which the present invention pertains will be able to understand that the present invention can be implemented in a modified form without departing from the essential characteristics of the present invention. Therefore, the disclosed embodiments should be considered from an illustrative rather than a limiting point of view. The scope of the present invention is shown in particular in the claims rather than the foregoing description, and all differences within the equivalent range will be construed as being included in the present invention.

## Claims

1. A pharmaceutical composition for preventing or treating neurodegenerative disease, comprising a compound represented by the following Chemical Formula 1 or a pharmaceutically acceptable salt thereof: 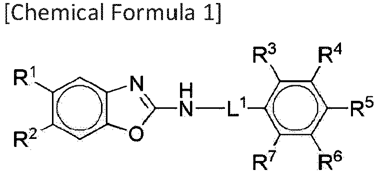 in Chemical Formula 1,
L¹ is a single bond or 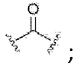
R¹ and R² are each -H, -OH, a C₁₋₁₀ straight or branched alkyl, or a C₆₋₈ arylamide, wherein the aryl of the arylamide is optionally substituted with one or more of a halogen, -NO₂ and a C₁₋₁₀ straight or branched alkyl halide;
R¹ and R² optionally form a C₆₋₈ aryl with a carbon atom to which they are linked; and
R³, R⁴, R⁵, R⁶ and R⁷ are each -H, a halogen, -NO₂ or a C₁₋₁₀ straight or branched alkyl).

2. The pharmaceutical composition of claim 1, wherein L¹ is a single bond or 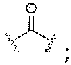 R¹ and R² are each -H, -OH, a C₁₋₅ straight or branched alkyl, or a C₆₋₇ arylamide, wherein the aryl of the arylamide is optionally substituted with one or more of a halogen, -NO₂ and a C₁₋₅ straight or branched alkyl halide; R¹ and R² optionally form a C₆₋₇ aryl with a carbon atom to which they are linked; and R³, R⁴, R⁵, R⁶ and R⁷ are each -H, a halogen, -NO₂ or a C₁₋₅ straight or branched alkyl.

3. The pharmaceutical composition of claim 1, wherein L¹ is a single bond or 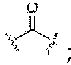 R¹ and R² are each -H, -OH, -CH₃, or phenylamide, wherein the phenyl of the phenylamide is optionally substituted with one or more of -Cl, -NO₂ and -CH₂Cl; R¹ and R² optionally form a phenyl with a carbon atom to which they are linked; and R³, R⁴, R⁵, R⁶ and R⁷ are each -H, -F, -Cl, -NO₂ or -CH₂CH₃.

4. The pharmaceutical composition of claim 1, wherein L¹ is a single bond or 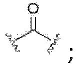 R¹ is -H, -OH, -CH₃, 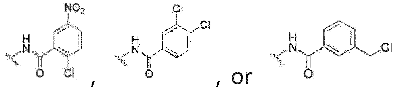 ; R² is -H; R¹ and R² optionally form a phenyl with a carbon atom to which they are linked; R³ is -H or -Cl; R⁴ is -H, -F or -Cl; R⁵ is -F, -Cl, -NO₂, or -CH₂CH₃; R⁶ is -H; and R⁷ is -H.

5. The pharmaceutical composition of claim 1, wherein the compound represented by Chemical Formula 1 is any one selected from the following compound group:
1) N-(benzo[d]oxazol-2-yl)-2-chloro-4-nitrobenzamide;
2) 8-methyl-2-[N-(3,4-dichlorophenyl)]aminobenzoxazole;
3) 2-((3,4-dichlorophenyl)amino)benzo[d]oxazol-5-ol;
4) N-(2-(4-ethylphenylamino)benzo[d]oxazol-5-yl)-2-chloro-5-nitrobenzamide;
5) N-(2-(4-ethylphenylamino)benzo[d]oxazol-5-yl)-3,4-dichlorobenzamide;
6) N-(2-(4-ethylphenylamino)benzo[d]oxazol-5-yl)-3-(chloromethyl)benzamide;
7) 2-[N-(3,4-dichlorophenyl)]aminobenzoxazole;
8) N-(3,4-dichlorophenyl)naphtho[2,3-d]oxazol-2-amine;
9) N-(3,4-difluorophenyl)-5-methylbenzo[d]oxazol-2-amine; and
10) N-(3,4-difluorophenyl)benzo[d]oxazol-2-amine.

6. The pharmaceutical composition of claim 1, wherein the composition increases the expression level of a klotho gene.

7. The pharmaceutical composition of claim 1, wherein the neurodegenerative disease comprises one or more diseases selected from the group consisting of stroke, amnesia, memory loss, memory impairment, dementia, forgetfulness, cognitive dysfunction, Parkinson's disease, Alzheimer's disease, Pick's disease, Creutzfeld-Kacob disease, Huntington's disease, and Lou Gehrig's disease.

8. A health functional food composition for preventing or improving neurodegenerative disease, comprising a compound represented by Chemical Formula 1 or a pharmaceutically acceptable salt thereof: 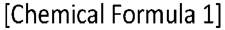 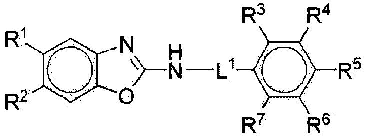 (in Chemical Formula 1,
L¹ is a single bond or 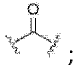
R¹ and R² are each -H, -OH, a C₁₋₁₀ straight or branched alkyl, or a C₆₋₈ arylamide, wherein the aryl of the arylamide is optionally substituted with one or more of a halogen, -NO₂ and a C₁₋₁₀ straight or branched alkyl halide;
R¹ and R² optionally form a C₆₋₈ aryl with a carbon atom to which they are linked; and
R³, R⁴, R⁵, R⁶ and R⁷ are each -H, a halogen, -NO₂ or a C₁₋₁₀ straight or branched alkyl).

9. A food composition for preventing or improving neurodegenerative disease, comprising a compound represented by Chemical Formula 1 or a pharmaceutically acceptable salt thereof: 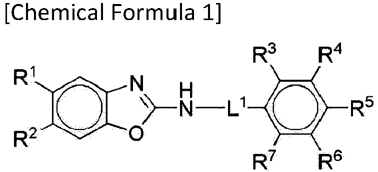 (in Chemical Formula 1,
L¹ is a single bond or 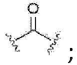
R¹ and R² are each -H, -OH, a C₁₋₁₀ straight or branched alkyl, or a C₆₋₈ arylamide, wherein the aryl of the arylamide is optionally substituted with one or more of a halogen, -NO₂ and a C₁₋₁₀ straight or branched alkyl halide;
R¹ and R² optionally form a C₆₋₈ aryl with a carbon atom to which they are linked; and
R³, R⁴, R⁵, R⁶ and R⁷ are each -H, a halogen, -NO₂ or a C₁₋₁₀ straight or branched alkyl).

10. A method for preventing or treating neurodegenerative disease comprising the step of administering or taking a composition comprising a compound represented by Chemical Formula 1 or a pharmaceutically acceptable salt thereof to a subject: 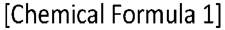 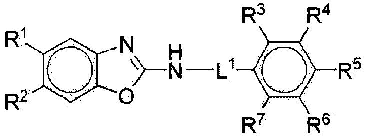 (in Chemical Formula 1,
L¹ is a single bond or 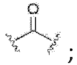
R¹ and R² are each -H, -OH, a C₁₋₁₀ straight or branched alkyl, or a C₆₋₈ arylamide, wherein the aryl of the arylamide is optionally substituted with one or more of a halogen, -NO₂ and a C₁₋₁₀ straight or branched alkyl halide;
R¹ and R² optionally form a C₆₋₈ aryl with a carbon atom to which they are linked; and
R³, R⁴, R⁵, R⁶ and R⁷ are each -H, a halogen, -NO₂ or a C₁₋₁₀ straight or branched alkyl).

11. Use of a composition comprising a compound represented by Chemical Formula 1 or a pharmaceutically acceptable salt thereof for preventing or treating neurodegenerative disease: 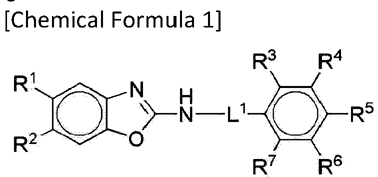 (in Chemical Formula 1,
L¹ is a single bond or 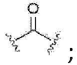
R¹ and R² are each -H, -OH, a C₁₋₁₀ straight or branched alkyl, or a C₆₋₈ arylamide, wherein the aryl of the arylamide is optionally substituted with one or more of a halogen, -NO₂ and a C₁₋₁₀ straight or branched alkyl halide;
R¹ and R² optionally form a C₆₋₈ aryl with a carbon atom to which they are linked; and
R³, R⁴, R⁵, R⁶ and R⁷ are each -H, a halogen, -NO₂ or a C₁₋₁₀ straight or branched alkyl).
